# EUROPEAN PATENT APPLICATION

(11) **EP 3 988 690 A1**
(43) Date of publication of application: **27.04.2022**
(21) Application number: 20826792.2
(22) Date of filing: 12.05.2020
(51) Int. Cl.: C40B 40/06, C12N 15/10, C12Q 1/686, C12Q 1/6869, C12Q 1/6876, C12Q 1/689, C12Q 1/6895

(54) **METHOD FOR PREPARING DNA LIBRARY**

(30) Priority: 21.06.2019 JP 2019115373
(71) Applicant: Asahi Group Holdings, Ltd., Tokyo 130-8602 (JP)
(72) Inventor: KURNIAWAN Yohanes Novi, Moriya-shi, Ibaraki 302-0106 (JP); SHINOHARA Yuji, Moriya-shi, Ibaraki 302-0106 (JP)
(74) Representative: Hoffmann Eitle
(86) International application number: PCT/JP2020/018992
(87) International publication number: WO 2020/255587

(57) **Abstract**

A method for preparing a DNA library for identifying a microbial species using a nanopore sequencer in a shorter time than before, and a method for identifying a microorganism contained in a test sample using the method are provided. The present invention provides a method for preparing a DNA library for identifying a microbial species using a nanopore sequencer, the method including a DNA extraction step of suspending microbial cells, which have been isolated and cultured, in an alkaline solution having a pH of 8.0 to 8.6, performing a heat treatment on the obtained suspension at 90°C to 100°C for 1 to 15 minutes, and performing a centrifugal treatment on the heat-treated suspension to collect a supernatant, a PCR step of performing hot start PCR using a polymerase which requires a time of 10 seconds or shorter for 1 kb extension, using DNA contained in the supernatant as a template, and an adapter addition step of adding an adapter, to which a motor protein is bound, to an end of a PCR product obtained in the PCR step.

## Description

### [Technical Field]

The present invention relates to a method for preparing a DNA library for identifying a microbial species using a nanopore sequencer.

Priority is claimed on Japanese Patent Application No. 2019-115373, filed on June 21, 2019, the content of which is incorporated herein by reference.

### [Background Art]

Processed foods and drinks are required to maintain the quality thereof from the time of the production until the time the processed foods and drinks are consumed by general consumers. In particular, since microorganisms cause not only quality degradation but also food poisoning, prevention of microorganism contamination is extremely important from the viewpoint of food safety. As a method for testing microorganisms in foods and drinks, a method for isolating microorganisms from foods and drinks serving as a test sample, culturing the microorganisms in an appropriate culture medium, and identifying the microorganisms based on the microbial properties is an exemplary example. In recent years, as a more rapid method for identifying microorganisms, a method for performing gene analysis on DNA of isolated and cultured microbial cells has been used.

In the microorganism identification using gene analysis, the base sequence of rDNA is generally used. The base sequence of rDNA extracted from a test sample is determined using a sequencer, and the microorganism species in the test sample is identified based on the information of the obtained base sequence with reference to international base sequence databases such as DDBJ, EMBL, and GenBank. In this case, in order to efficiently detect a trace amount of microorganisms present in the test sample, a specific region of rDNA is amplified in advance by PCR (polymerase chain reaction) or the like, and the obtained amplification product is provided for a sequencer.

In the microorganism testing performed in a factory or the like for producing processed foods and drinks, rapidity is required so as not to impair the freshness of the processed foods and drinks. It is preferable that the microorganism testing be performed in a factory so that the testing can be performed more rapidly, but genetic testing usually requires a large sequencer. In general, the testing is performed by carrying out the isolation of microorganisms from a test sample up to the collection of genomic DNA in a factory and sending the collected genomic DNA to a facility where genetic testing can be performed or sending a processed food or drink serving as a test sample from the factory to a facility where genetic testing can be performed. In recent years, among third-generation sequencers, nanopore sequencers that identify the base sequence based on a change in current generated in a case where DNA passes through nanopores (nanoscale holes) have been developed (for example, see Non-Patent Document 1). In particular, an extremely small-sized nanopore sequencer "MinION" (manufactured by Oxford Nanopore Technologies Ltd.) is a portable sequencer that enables gene analysis in various places.

### [Citation List]

### [Non-Patent Document]

### [Non-Patent Document 1]

Anonymous, Nature Biotechnology, 2018, vol. 36, p 287

### [Summary of Invention]

### [Technical Problem]

It is expected that genetic analysis can be performed directly in each factory and the presence or absence of microorganism contamination of processed foods and drinks to be tested can be analyzed more quickly than before, by using a portable sequencer such as "MinION". However, in order to provide a DNA library for a nanopore sequencer, it is necessary to perform PCR amplification on a target genomic fragment for identifying the base sequence after extraction of DNA from the test sample to prepare a DNA library in which an adapter is added to the obtained amplification product. However, it takes nearly 5 hours to prepare the DNA library, and thus a further reduction in time is required. In the microorganism identification method widely used for nanopore sequencers, bacteria can be identified with a certain degree of accuracy, but fungi such as yeasts are difficult to identify.

An object of the present invention is to provide a method for preparing a DNA library for identifying a microbial species using a nanopore sequencer in a shorter time than before, and a method for identifying a microorganism contained in a test sample using the method.

### [Solution to Problem]

The present inventors found that in the preparation of a DNA library to be provided for a nanopore sequencer, the time for preparing a DNA library can be significantly reduced by performing an alkaline heat treatment in order to extract DNA from the test sample and performing hot start PCR as PCR using the extracted DNA as a template, thereby completing the present invention.

That is, the present invention is as follows.
[1] A method for preparing a DNA library for identifying a microbial species using a nanopore sequencer, the method including: a DNA extraction step of suspending microbial cells, which have been isolated and cultured, in an alkaline solution having a pH of 8.0 to 8.6, performing a heat treatment on the obtained suspension at 90°C to 100°C for 1 to 15 minutes, and performing a centrifugal treatment on the heat-treated suspension to collect a supernatant; a PCR step of performing hot start PCR using a polymerase which requires a time of 10 seconds or shorter for 1 kb extension using DNA contained in the supernatant as a template; and an adapter addition step of adding an adapter, to which a motor protein is bound, to an end of a PCR product obtained in the PCR step.
[2] The method for preparing a DNA library according to [1], in which the hot start PCR is performed such that one cycle consisting of heat denaturation, annealing, and extension is carried out within 70 seconds.
[3] The method for preparing a DNA library according to [1] or [2], in which a PCR product of 700 bp or longer is obtained in the hot start PCR.
[4] The method for preparing a DNA library according to any one of [1] to [3], in which the hot start PCR is performed using:
   (1) a set consisting of a forward primer represented by SEQ ID NO: 1 and a reverse primer represented by SEQ ID NO: 2,
   (2) a set consisting of a forward primer consisting of a base sequence in which a barcode sequence represented by any of SEQ ID NOs: 3 to 14 is linked to a 3' end of a base sequence represented by SEQ ID NO: 15 and a base sequence represented by SEQ ID NO: 1 is linked to a 3' end of the barcode sequence and a reverse primer consisting of a base sequence in which the barcode sequence is linked to the 3' end of the base sequence represented by SEQ ID NO: 15 and a base sequence represented by SEQ ID NO: 2 is linked to the 3' end of the barcode sequence,
   (3) a set consisting of a forward primer represented by SEQ ID NO: 16 and a reverse primer represented by SEQ ID NO: 17, 18, or 19, or
   (4) a set, for 1st PCR, consisting of a forward primer consisting of a base sequence in which a base sequence represented by SEQ ID NO: 16 is linked to a 3' end of a base sequence represented by SEQ ID NO: 20 and a reverse primer consisting of a base sequence in which a base sequence represented by SEQ ID NO: 17 is linked to a 3' end of the base sequence represented by SEQ ID NO: 21 and
      a set, for 2nd PCR, consisting of a forward primer consisting of a base sequence in which the barcode sequence represented by any of SEQ ID NOs: 3 to 14 is linked to the 3' end of the base sequence represented by SEQ ID NO: 15 and the base sequence represented by SEQ ID NO: 20 is linked to the 3' end of the barcode sequence and a reverse primer consisting of a base sequence in which the barcode sequence represented by any of SEQ ID NOs: 3 to 14 is linked to the 3' end of the base sequence represented by SEQ ID NO: 15 and the base sequence represented by SEQ ID NO: 21 is linked to the 3' end of the barcode sequence.
[5] The method for preparing a DNA library according to any one of [1] to [4], in which in the adapter addition step, the end of the PCR product is repaired to perform polyadenylation of a 3' end of the PCR product without measurement of the spectrometric absorbance, and a poly(T) adapter is ligated and added to the polyadenylated PCR product.
[6] The method for preparing a DNA library according to any one of [1] to [4], in which in the adapter addition step, the PCR product is purified using magnetic beads each having a surface modified with a carboxyl group, and an adapter is added to an end of the PCR product without measurement of the spectrometric absorbance of the purified DNA.
[7] The method for preparing a DNA library according to any one of [1] to [6], in which the microbial cell is a fungus or a bacterium.
[8] The method for preparing a DNA library according to any one of [1] to [7], in which a process from start of the DNA extraction step to end of the adapter addition step is performed within 200 minutes.
[9] A method for identifying a microbial species of a microorganism, including:
   preparing a DNA library from microbial cells individually isolated and cultured from a test sample using the method for preparing a DNA library according to any one of [1] to [8]; analyzing the DNA library with a nanopore sequencer; and identifying the microbial species of the microbial cells based on obtained base sequence output data.
[10] A method for identifying a microbial species of a microorganism, including: preparing a DNA library from microbial cells individually isolated and cultured from a test sample using the method for preparing a DNA library according to any one of [1] to [8]; analyzing the DNA library with a nanopore sequencer; determining a consensus base sequence based on obtained base sequence output data; and identifying a microbial species of the microbial cells based on the determined consensus base sequence.
[11] A method for evaluating microorganism contamination in a processed food or drink, the method comprising: preparing a processed food or drink or a semi-finished product collected in the course of a production process of the processed food or drink as a test sample; isolating and culturing microorganisms contained in the test sample and identifying a microbial species of each microorganism using the method for identifying a microbial species of a microorganism according to [9] or [10]; evaluating that a semi-finished product and a finished product in the same production lot as in the test sample are contaminated by a contamination-causing microorganism in a case where the test sample contains a contamination-causing microorganism; and evaluating that a semi-finished product and a finished product in the same production lot as in the test sample are not contaminated by a contamination-causing microorganism in a case where the test sample does not contain a contamination-causing microorganism.
[12] The method for evaluating microorganism contamination according to [11], in which the test sample is a processed food or drink before being shipped from a factory, in a case where the test sample contains a contamination-causing microorganism, it is evaluated that the processed food or drink in the same production lot as in the test sample is not shippable, and in a case where the test sample does not contain a contamination-causing microorganism, it is evaluated that the processed food or drink in the same production lot as in the test sample is shippable.
[13] The method for evaluating microorganism contamination according to [11], in which in a case where the test sample contains a contamination-causing microorganism, it is evaluated that the production line from which the test sample has been collected is contaminated with the contamination-causing microorganism, and in a case where the test sample does not contain a contamination-causing microorganism, it is evaluated that the production line from which the test sample has been collected is not contaminated with the contamination-causing microorganism.
[14] The method for evaluating microorganism contamination according to any one of [11] to [13], in which the processed food or drink is a packaged beer taste beverage.
[15] The method for evaluating microorganism contamination according to [14], in which the processed food or drink is a packaged beer taste beverage that has not been subjected to a heat sterilization treatment after being packaged, and in a case where the test sample does not contain a contamination-causing microorganism, it is evaluated that the packaged beer taste beverage in the same production lot as in the test sample is shippable as a non-heat-treated beer taste beverage.

### [Advantageous Effects of Invention]

With the method for preparing a DNA library according to the present invention, the time for preparing a DNA library can be significantly reduced as compared with a method for preparing a DNA library of the related art, such as a method recommended by a manufacturer and a distributor of a nanopore sequencer. Therefore, microorganisms in the test sample can be rapidly identified by using the preparation method.

### [Description of Embodiments]

A method for preparing a DNA library according to the present invention is a method for preparing a DNA library for identifying a microbial species using a nanopore sequencer. The prepared DNA library is an analysis sample to be provided for a nanopore sequencer. In the nanopore sequencer, it is necessary to bind a motor protein to a double-stranded DNA fragment to be analyzed in advance. In a case where the motor protein is bound to a nanopore protein on the membrane, the motor protein unwinds the double-stranded DNA to which the motor protein is bound into a single strand, and sends the single-stranded DNA to pass through the nanopore. The current generated when passing through this nanopore is different for each of the four bases (AGCT). Therefore, the base sequence of the single-stranded DNA that has passed through the nanopore is identified based on this change in current. Therefore, it is necessary to link a DNA fragment as an adapter, to which a motor protein can be bound, to the double-stranded DNA to be analyzed by a nanopore sequencer in advance. That is, the DNA library to be provided for a nanopore sequencer is a group of conjugates of double-stranded DNA having an end to which an adapter and motor proteins are added.

Specifically, a DNA library for identifying a microbial species using a nanopore sequencer is prepared by performing a DNA extraction step of extracting DNA from microbial cells in which microorganisms to be identified have been cultured, a PCR step of amplifying the base sequence of double-stranded DNA to be analyzed, using extracted DNA as a template by PCR, and an adapter addition step of adding an adapter to an end of the obtained PCR product. Hereinafter, each step will be described.

A DNA library prepared by the method for preparing a DNA library according to the present invention is formed by binding an adapter and a motor protein to an amplification product obtained by amplifying the base sequence of targeted analysis region from the genomic DNA of the microorganism strain to be identified by PCR. The base sequence analysis using a nanopore sequencer tends to have lower analysis accuracy than that of the analysis using a so-called first-generation or second-generation sequencer. Therefore, in order to identify the microbial species with a sufficient level of accuracy, the DNA library is prepared from the genomic DNA of the isolated and cultured microbial cells. Specifically, the DNA library to be prepared in the present invention is prepared from the genomic DNA such as microbial cells constituting 1 to 12 colonies formed by culturing the test sample expected to contain a plurality of microorganisms as in a case of a processed food or drink in a plate medium according to a plate smear method and microbial cells obtained by liquid-culturing isolated microorganism strains. The target microorganism for identifying a biological species may be a bacterium or a fungus such as a yeast or a mold.

In the DNA extraction step, the isolated and cultured microbial cells are suspended in an alkaline solution having a pH of 8.0 to 8.6, and the obtained suspension is subjected to a heat treatment at 90°C to 100°C for 1 to 15 minutes and subjected to a centrifugal treatment to collect the supernatant. In the related art, in the preparation of a DNA library to be provided for a nanopore sequencer, in order to increase the identification accuracy of microorganisms, the extraction of genomic DNA from microbial cells is performed by sequentially performing lysozyme treatment, proteinase K treatment and RNase treatment, and collecting DNA extracted from the microbial cells using magnetic beads. The method enables collection of DNA having high purification purity, but the method requires a multi-step process and thus takes about 120 minutes. Meanwhile, in the present invention, the microbial cells are subjected to an alkaline heat treatment, and the microbial cell residues are allowed to be precipitated by carrying out a centrifugal treatment to collect the supernatant containing DNA extracted from the microbial cells. In this manner, genomic DNA can be collected from the microbial cells in a significantly shorter time than before.

The alkaline solution used for the alkaline heat treatment performed on the microbial cells is not particularly limited as long as the alkaline solution is a solution typically used for extraction of DNA from microorganisms and cultured cells and having a pH of 8.0 to 8.6, and can be appropriately selected from the alkaline solutions used in the technical field and used. Further, it is preferable to use a commercially available DNA extraction kit or the like. In the present invention, it is preferable to use an alkaline solution having a small content or no content of a component having a negative impact on PCR. In a case where such a solution is used, since the obtained DNA extraction liquid can be used as a template solution for PCR as it is or only by being diluted 2- to 6-fold with water, the step of purifying DNA from the DNA extraction liquid is not necessary, and thus this results in a reduction of the time. As such an alkaline solution, for example, "Prepman (registered trademark) Ultra reagent" (manufactured by Thermo Fisher Scientific)" is preferably used.

Next, the PCR step is carried out by performing hot start PCR using DNA contained in the supernatant collected in the DNA extraction step as a template, and using a polymerase which requires a time of 10 seconds or shorter for 1 kb extension (10 sec/kb or less). The nanopore sequencer is a long-read sequencer, and a relatively long-chain PCR product, for example, a PCR product of 700 bp or longer is amplified in the PCR step. In the present invention, in a case of using a polymerase having an extension rate of 10 sec/kb or less, which is extremely fast, each reaction time for heat denaturation, annealing, and extension can be set to 30 seconds or shorter and preferably 20 seconds or shorter even in a case where a PCR product of 700 bp or longer is amplified, and thus a PCR product can be obtained in an extremely short time. Particularly in a case of using a thermal cycler having a high temperature increase/decrease rate, one cycle consisting of heat denaturation, annealing, and extension can be performed in, for example, 65 to 70 seconds, and the PCR product can be amplified in approximately 35 to 45 minutes. Further, in the present invention, a PCR product with high amplification accuracy can be obtained by performing hot start PCR. Further, the reaction conditions for PCR can be appropriately determined in consideration of the base pair length of the PCR product to be amplified, the kind of DNA polymerase to be used, the Tm value of the primer to be used, and the like.

The polymerase used in the present invention is not particularly limited as long as the polymerase has an extension rate of 10 sec/kb or less, is inactivated before the heat treatment, and is a DNA polymerase that recovers the polymerase activity by the heat treatment. Further, the polymerase can be used by being appropriately selected from the DNA polymerases which have been used in hot start PCR, and a commercially available DNA polymerase for hot start PCR or a kit including the same is preferably used. Preferred examples of the DNA polymerase for hot start PCR with an extension rate of 10 sec/kb include "SapphireAmp (registered trademark) Fast PCR Master Mix" (manufactured by Takara Bio Inc.) and "Q5 High-Fidelity DNA Polymerase" (manufactured by New England Biolabs).

As the primer set used for PCR, a primer set that targets and amplifies a partial region of rDNA in genomic DNA, which is a region having high homology (sequence identity) of the base sequence between microorganism species is used. The target region to be amplified and the primer to be used can be designed using a commonly used primer design tool based on, for example, the base sequence information of rDNA of a microorganism group including the microorganism to be identified and can be synthesized by a known DNA synthesis method.

In the present invention, in a case where genomic DNA prepared from a single kind of microorganism strain is used as a template and the identification target is a bacterium, for example, the identification can be performed using a set of Ps1-forward primer and Ps1-reverse primer listed in Table 1. In a case where the identification target is a fungus such as a yeast, for example, the identification can be performed using a set of the Es1-forward primer and the Es1-reverse primer, a set of the Es1-forward primer and the Es2-reverse primer, or a set of the Es1-forward primer and the Es3reverse primer listed in Table 2.

**[Table 1]**

| For single strain of bacterium | Base sequence (5'→3') | Target gene | Amplification length | SEQ ID NO |
|---|---|---|---|---|
| Ps1-forward primer | AGAGTTTGATCMTGGCTCAG | 27th of 16S rDNA | 1466bp | 1 |
| Ps1-reverse primer | GGTTACCTTGTTACGACTT | 1492nd of 16S rDNA | | 2 |

**[Table 2]**

| For single strain of fungus | Base sequence (5'→3') | Target gene | Amplification length | SEQ ID NO |
|---|---|---|---|---|
| Es1-forward primer | TGTACACACCGCCCGTCG | 18S rDNA | 700-1000bp | 16 |
| Es1-reverse primer | GAGCTGCATTCCCAAACAACTC | 26S/28S rDNA | | 17 |
| Es1-forward primer | TGTACACACCGCCCGTCG | 18S rDNA | 1200-1300bp | 16 |
| Es2-reverse primer | AATGCATTGGTCCGTGTTTCAAGACG | 26S/28S rDNA | | 18 |
| Es1-forward primer | TGTACACACCGCCCGTCG | 18S rDNA | 1300-1400bp | 16 |
| Es3-reverse primer | CTTGGTCCGTGTTTCAAGAC | 26S/28S rDNA | | 19 |

In a case where genomic DNA prepared from 2 to 12 kinds of microorganism strains is used as a template, primers having 12 kinds of barcode sequences listed in Table 3 can be used.

**[Table 3]**

| Barcode sequence | Base sequence (5'→3') | SEQ ID NO |
|---|---|---|
| 16S01 | AAGAAAGTTGTCGGTGTCTTTGTG | 3 |
| 16S02 | TCGATTCCGTTTGTAGTCGTCTGT | 4 |
| 16S03 | GAGTCTTGTGTCCCAGTTACCAGG | 5 |
| 16S04 | TTCGGATTCTATCGTGTTTCCCTA | 6 |
| 16S05 | CTTGTCCAGGGTTTGTGTAACCTT | 7 |
| 16S06 | TTCTCGCAAAGGCAGAAAGTAGTC | 8 |
| 16S07 | GTGTTACCGTGGGAATGAATCCTT | 9 |
| 16S08 | TTCAGGGAACAAACCAAGTTACGT | 10 |
| 16S09 | AACTAGGCACAGCGAGTCTTGGTT | 11 |
| 16S10 | AAGCGTTGAAACCTTTGTCCTCTC | 12 |
| 16S11 | GTTTCATCTATCGGAGGGAATGGA | 13 |
| 16S12 | CAGGTAGAAAGAAGCAGAATCGGA | 14 |

In the present invention, in a case where genomic DNAs prepared from 2 to 12 kinds of bacterium strains are used as a template, for example, the identification can be performed using a set of Pm1-forward primer and Pm1-reverse primer listed in Table 4. Further, in the table, the "barcode" indicates any of the 12 kinds of barcode sequences listed in Table 3. That is, the primer set of the Pm1-forward primer and the Pm1-reverse primer listed in Table 4 is a set consisting of 12 kinds of forward primers (Pm1-forward primer) consisting of the base sequence in which the barcode sequence represented by any of SEQ ID NOs: 3 to 14 is linked to the 3' end of the base sequence represented by SEQ ID NO: 15 (ATCGCCTACCGTGAC) and the Ps1-forward primer (the base sequence represented by SEQ ID NO: 1) is linked to the 3' end of the barcode sequence and 12 kinds of reverse primers (Pml-reverse primer) consisting of the base sequence in which the barcode sequence is linked to the 3' end of the base sequence represented by SEQ ID NO: 15 and the Ps1-reverse primer (the base sequence represented by SEQ ID NO: 2) is linked to the 3' end of the barcode sequence.

**[Table 4]**

| For plural strains | Base sequence (5'→3') |
|---|---|
| Pm1-forward primer | ATCGCCTACCGTGAC-barcode-AGAGTTTGATCMTGGCTCAG |
| Pm1-reverse primer | ATCGCCTACCGTGAC-barcode-GGTTACCTTGTTACGACTT |
| Em1-forward primer | ATCGCCTACCGTGAC-barcode-TGTACACACCGCCCGTCG |
| Em1-reverse primer | ATCGCCTACCGTGAC-barcode-CTTGGTCCGTGTTTCAAGAC |

In the present invention, in a case where genomic DNAs prepared from a group consisting of approximately 30 kinds of microorganism strains each containing fungi and bacteria are used as a template, for example, the identification can be performed using a set of the Pm1-forward primer and the Pm1-reverse primer and a set of the Em1-forward primer and the Em1-reverse primer listed in Table 4. The primer set of the Em1-forward primer and the Em1-reverse primer listed in Table 4 is a set consisting of 12 kinds of forward primers (Em1-forward primer) consisting of the base sequence in which the barcode sequence represented by any of SEQ ID NOs: 3 to 14 is linked to the 3' end of the base sequence represented by SEQ ID NO: 15 and the Es1-forward primer (the base sequence represented by SEQ ID NO: 16) is linked to the 3' end of the barcode sequence and 12 kinds of reverse primers (Eml-reverse primer) consisting of the base sequence in which the barcode sequence is linked to the 3' end of the base sequence represented by SEQ ID NO: 15 and the Es3-reverse primer (the base sequence represented by SEQ ID NO: 19) is linked to the 3' end of the barcode sequence.

In the present invention, in a case where genomic DNAs prepared from a group consisting of 2 to 12 kinds of microorganism strains containing fungi and bacteria are used as a template, two-step PCR can be performed. 1st PCR can be performed using, for example, a set of the Em2-forward primer and the Em2-reverse primer listed in Table 5 for DNA prepared from fungi and using, for example, a set of primers listed in Table 6 for DNA prepared from bacteria. 2nd PCR can be performed using a set of the Em3-forward primer and the Em3-reverse primer listed in Table 7. Here, the Em2-forward primer is a primer consisting of the base sequence (SEQ ID NO: 22) in which Es1-forward primer (the base sequence represented by SEQ ID NO: 16) is linked to the 3' end of the base sequence represented by SEQ ID NO: 20
(TTTCTGTTGGTGCTGATATTGC) and the Em2-reverse primer is a primer consisting of the base sequence (SEQ ID NO: 23) in which Es1-reverse primer (the base sequence represented by SEQ ID NO: 17) is linked to the 3' end of the base sequence represented by SEQ ID NO: 21 (ACTTGCCTGTCGCTCTATCTTC). A primer obtained by replacing the Es1-reverse primer in the Em2-reverse primer with the Es2-reverse primer (SEQ ID NO: 18) or the Es3-reverse primer (SEQ ID NO: 19) can also be used in place of the Em2-reverse primer. Further, the Pm2-forward primer is a primer consisting of the base sequence (SEQ ID NO: 24) in which the Ps1-forward primer (the base sequence represented by SEQ ID NO: 1) is linked to the 3' end of the base sequence represented by SEQ ID NO: 20 and the Pm2-reverse primer is a primer consisting of the base sequence (SEQ ID NO: 25) in which the Ps1-reverse primer (the base sequence represented by SEQ ID NO: 2) is linked to the 3' end of the base sequence represented by SEQ ID NO: 21.

Further, in the table, the "barcode" indicates any of the 12 kinds of barcode sequences listed in Table 3. That is, the primer set listed in Table 7 is a set consisting of 12 kinds of forward primers (Em3-forward primer) consisting of the base sequence in which the barcode sequence represented by any of SEQ ID NOs: 3 to 14 is linked to the 3' end of the base sequence represented by SEQ ID NO: 15 (ATCGCCTACCGTGAC) and the base sequence represented by SEQ ID NO: 20 is linked to the 3' end of the barcode sequence and 12 kinds of reverse primers (Em3-reverse primer) consisting of the base sequence in which the barcode sequence is linked to the 3' end of the base sequence represented by SEQ ID NO: 15 and the base sequence represented by SEQ ID NO: 21 is linked to the 3' end of the barcode sequence.

**[Table 5]**

| For plural strains of fungus | Base sequence (5'→3') | SEQ ID NO |
|---|---|---|
| Em2-forward primer | TTTCTGTTGGTGCTGATATTGC-TGTACACACCGCCCGTCG | 22 |
| Em2-reverse primer | ACTTGCCTGTCGCTCTATCTTC-GAGCTGCATTCCCAAACAACTC | 23 |

**[Table 6]**

| For plural strains of bacterium | Base sequence (5'→3') | SEQ ID NO |
|---|---|---|
| Pm2-forward primer | TTTCTGTTGGTGCTGATATTGC-AGAGTTTGATCMTGGCTCAG | 24 |
| Pm2-reverse primer | ACTTGCCTGTCGCTCTATCTTC-GGTTACCTTGTTACGACTT | 25 |

**[Table 7]**

| For plural strains | Base sequence (5'→3') |
|---|---|
| Em3-forward primer | ATCGCCTACCGTGAC-barcode-TTTCTGTTGGTGCTGATATTGC |
| Em3-reverse primer | ATCGCCT ACCGTGAC-barcode-ACTTGCCTGTCGCTCTATCTTC |

Next, the adapter addition step is performed by adding an adapter to the end of the PCR product obtained in the PCR step. The adapter is not particularly limited as long as the adapter is an oligonucleotide that can bound to the motor protein to be used. For example, a complex of two kinds of oligonucleotides listed in Table 8 can be used as the adapter. The underlined part in the Adapter Y top fragment is a region consisting of a base sequence complementary to the Adapter Y bottom fragment and hybridizes with the Adapter Y bottom fragment. The motor protein is bound to the 5' end side of the Adapter Y top fragment of the adapter. As the motor protein, phage phi29 polymerase, helicase, or the like can be used.

**[Table 8]**

| Adapter | Base sequence (5'→3') | SEQ ID NO |
|---|---|---|
| Adapter Y top | GGCGTCTGCTTGGGTGTTTAACCTTTTTTTTTAATGTACTT CGTTCAGTTACGTATTGCT | 26 |
| Adapter Y bottom | GCAATACGTAACTGAACGAAGT | 27 |

The adapter can be added to the PCR product using a commercially available reagent or the like according to, for example, a method recommended by the manufacturer of the nanopore sequencer "MinION" (manufactured by Oxford Nanopore Technologies Ltd.).

Specifically, in a case of a PCR product obtained using the primer set listed in Table 1 or Table 2, the end of the PCR product is repaired to perform polyadenylation of the 3' end, and all the Adapter Y top, the Adapter Y bottom, and the motor protein are added together with T4 DNA ligase to carry out a ligation reaction. The Adapter Y top is a poly(T) adapter, and the adapter is added to the 3' end of the polyadenylated PCR product as a result of linking the poly T region and the poly A region of the polyadenylated PCR product to each other. The repair of the end of the PCR product and the polyadenylation of the 3' end can be carried out by a method of the related art using various commercially available enzymes, kits, and the like used for the repair of the end of the PCR product. For example, the repair and the polyadenylation of the end of the PCR product can be performed by using NEBNext FFPE DNA Repair Mix (manufactured by New England Biolabs) and Ultra II End-prep enzyme mix (manufactured by New England Biolabs) at the same time among commercially available kits.

It is preferable that the PCR product to which the adapter has been added be purified before being provided for a nanopore sequencer. The purification can be performed by being appropriately selected from known nucleic acid purification methods such as a method for adsorbing the PCR product on magnetic beads each having a surface modified with a carboxyl group and eluting the product. As the magnetic beads, for example, commercially available magnetic beads for nucleic acid purification can be used.

In a case where a plurality of the microbial species of microbial strains is identified, the adapter addition reaction can be carried out on the PCR product of 2 to 6 kinds of microbial strains with one reaction solution in the adapter addition step. However, the process before the adapter addition step is individually performed on each microorganism strain. For example, DNA is individually extracted from the microbial cells obtained by being individually isolated and cultured for 2 to 12 kinds of isolated microorganism strains, and hot start PCR is carried out using any of the primer sets listed in Tables 4 to 7 using each DNA as a template individually. The adapter addition step is performed on a mixture obtained by mixing equal amounts of the PCR products obtained from each microorganism strain. In a case of the PCR product obtained by using any of the primer sets listed in Tables 4 to 7, the Adapter Y top, the Adapter Y bottom, and the motor protein can be allowed to directly come into contact with each other and added to the PCR product without repairing the end of the PCR product. In this case, it is preferable that the PCR product be purified before the addition of the adapter. The purification can be performed by being appropriately selected from known nucleic acid purification methods such as a method for adsorbing the PCR product on magnetic beads each having a surface modified with a carboxyl group and eluting the product.

Typically, DNA concentration of the PCR product is measured, and the concentration is adjusted such that the amount of the PCR product is suitable for the subsequent reactions. At this time, for the sake of convenience, DNA concentration of the PCR product is acquired by, for example, measuring the spectrometric absorbance at 260 nm and calculating the concentration based on the obtained spectrometric absorbance value. However, in the method for preparing a DNA library according to the present invention, it is preferable that the obtained PCR product not be quantified after the completion of the PCR step. Specifically, in the case of the PCR product obtained using genomic DNA prepared from a single kind of microorganism strain as a template, the end of the obtained PCR product is directly repaired and the polyadenylation reaction of the 3' end is carried out without the measurement of the spectrometric absorbance. In a case of the PCR product obtained using genomic DNA prepared from two or more kinds of microorganism strains as a template, the DNA is purified using magnetic beads each having a surface modified with a carboxyl group, and the Adapter Y top, the Adapter Y bottom, and the motor protein are added without the measurement of the spectrometric absorbance of the purified DNA and added to the end of the purified PCR product. A DNA library can be prepared in a shorter time by not measuring the spectrometric absorbance for quantification.

According to the method for preparing the DNA library according to the present invention, the process from the start of the DNA extraction step to the end of the adapter addition step can be performed in 90 to 120 minutes by optimizing the method of extraction of DNA from microbial cells and the PCR method and further omitting the measurement of the spectrometric absorbance of the PCR product. The method for preparing a DNA library recommended by the manufacturer of the nanopore sequencer "MinION" (manufactured by Oxford Nanopore Technologies Inc.) requires a time of 260 to 290 minutes or longer to prepare a DNA library, but the method for preparing a DNA library according to the present invention enables a reduction in the time required to prepare a DNA library by 170 to 180 minutes or longer as compared with a method of the related art. Further, it is preferable that the prepared DNA library be stored on ice or in a low-temperature environment of approximately 4°C until the DNA library is provided for a nanopore sequencer.

The DNA library prepared by the method for preparing a DNA library according to the present invention can be directly provided for a nanopore sequencer. It is preferable that the nanopore sequencer "MinION" (manufactured by Oxford Nanopore Technologies Inc.) be used as the nanopore sequencer. In addition, loading of a DNA library sample for "MinION" and outputting of base sequence data of the individual DNA passing through the nanopores can be performed by the method recommended by the manufacturer of "MinION".

The base sequence data (base sequence output data) determined and output from the change in current when passing through the nanopores in the nanopore sequencer may have insufficient accuracy in sequence measurement. In this case, the consensus base sequence is determined from the base sequence output data. By identifying the microbial species based on the obtained consensus base sequence, the microbial species can be identified with higher accuracy than in a case where the identification of microbial species is attempted directly from the base sequence output data. In particular, in a case of identification of the microbial species such as a fungus, a consensus base sequence is preferably used. The consensus base sequence can be determined from the base sequence output data using, for example, an assembly function. As the assembly function, the assembly function of known sequence information analysis software such as "Geneious" (manufactured by Tomy Digital Biology Co., Ltd.) can be used.

The microbial species can be identified directly from the base sequence output data using the method recommended by the manufacturer of "MinION", but the identification accuracy of a fungus such as a yeast is extremely low with this method. Meanwhile, by identifying the microbial species based on the consensus base sequence determined by using the assembly function, the microbial species can be identified with high accuracy even in a case where the microbial species is a fungus such as a yeast, and the method can be applied to the microorganism testing for processed foods and drinks and the like. In particular, in the method for identifying a microbial species of a microorganism according to the present invention, that is, in the method for identifying a microbial species in which a DNA library is prepared by the method for preparing a DNA library according to the present invention, the obtained DNA library is analyzed using a portable nanopore sequencer "MinION", and the microbial species identified based on the consensus base sequence determined from the obtained base sequence output data is useful for microorganism testing at sites such as production factories for processed foods and drinks, from the viewpoints of rapidity and simplicity. The presence or absence of microorganism contamination in products, semi-finished products, and production factories can be evaluated by performing the method for identifying a microbial species of a microorganism according to the present invention on semi-finished products and finished products in production factories for processed foods and drinks, and cleaning liquids and smears in production lines. Further, the semi-finished product indicates an unfinished product in the course of the production.

For example, in production factories for processed foods and drinks, a processed food or drink which is a finished product or a semi-finished product collected in the course of the production process thereof is used as a test sample, microorganisms contained in the test sample are isolated and cultured, and the microbial species of each microorganism is identified by the method for identifying the microbial species of the microorganism according to the present invention. In a case where microorganisms that cause microorganism contamination in processed foods and drinks are defined as a contamination-causing microorganism and the test sample contains the contamination-causing microorganism, it is evaluated that semi-finished products and finished products in the same production lot as in the test sample are contaminated by the contamination-causing microorganism. On the contrary, in a case where the test sample does not contain the contamination-causing microorganism, it is evaluated that the semi-finished products and finished products in the same production lot as in the test sample are not contaminated by the contamination-causing microorganism. The semi-finished product is not particularly limited as long as the semi-finished product is a product at any point in the course of the production process. For example, in a case of a beer taste beverage packaged in a processed food or drink container, it is preferable that the semi-finished product used as a test sample be a fermenting liquid during a fermentation step, a fermentating liquid during a post-fermentation (aging) step, or the like.

For example, in a case where a processed food or drink before being shipped from a factory is used as a test sample, a determination as to whether to ship a product can be made. Specifically, in a case where the test sample contains a contamination-causing microorganism, it is evaluated that processed foods and drinks in the same production lot as in the test sample cannot be shipped. On the contrary, in a case where the test sample does not contain a contamination-causing microorganism, it is evaluated that the processed foods and drinks in the same production lot as in the test sample can be shipped. By performing the microorganism testing on processed foods and drinks before shipment using the method for identifying a microbial species of a microorganism according to the present invention, it is possible to investigate the presence or absence of microorganism contamination of a product far faster than before. Therefore, it is expected that a determination as to whether to ship a product can be quickly made.

Further, in a case where a processed food or drink shipped from a factory is used as a test sample, the safety of the processed food or drink in the distribution process can be evaluated. Specifically, in a case where the test sample does not contain a contamination-causing microorganism, processed foods and drinks in the same production lot as in the test sample can be evaluated as safe without the problem of microorganism contamination. On the contrary, in a case where the test sample contains a contamination-causing microorganism, processed foods and drinks in the same production lot as in the test sample can be evaluated as having a risk of microorganism contamination. According to the method for identifying a microbial species of a microorganism according to the present invention, the risk of microorganism contamination in processed foods and drinks after shipment can be rapidly evaluated, and necessary measures such as recall can be rapidly taken.

In addition, the microorganism contamination in finished products and semi-finished products may be caused by contamination of the lines in the production factories. Therefore, the evaluation of microorganism contamination of finished products and semi-finished products is also useful for determining the degree of contamination of the production factories. For example, in a case where the test sample contains a contamination-causing microorganism, it is evaluated that the production line from which the test sample has been collected is contaminated with the contamination-causing microorganism. On the contrary, in a case where the test sample does not contain a contamination-causing microorganism, it is evaluated that the production line from which the test sample has been collected is not contaminated with the contamination-causing microorganism.

In a case where the processed food or drink is a packaged beer taste beverage, examples of the bacteria among contamination-causing microorganisms in beer taste beverages include Lactobacillus spp. such as Lactobacillus brevis, Lactobacillus lindneri,

Lactobacillus paracollinoides, Lactobacillus paracasei, and Lactobacillus plantarum, Pediococcus spp. such as Pediococus claussenii and Pediococcus damnosus, Pectinatus spp. such as Pectinatus frisingensis and Pectinatus cerevisiiphilus, and Megasphaera spp. such as Megasphaera cerevisiae. Examples of the yeasts among contamination-causing microorganisms in beer taste beverages include wild strains of the genus Saccharomyces, Dekkera spp. such as Dekkera bruxellensis and Dekkera anomala, and Zygosaccharomyces sp. (also referred to as Zygosaccharomyces bisporus or Zygosaccharomyces parabailli).

Further, the "beer taste beverage" indicates a beer-like beverage. In addition, the term "beer-like" indicates a taste reminiscent of beer in terms of flavor, regardless of the product name or the label. That is, the beer taste beverage indicates a beverage that has a flavor, a taste, and a texture which are equivalent to or similar to those of beer, is highly thirst-quenching, and has drinkability, regardless of the alcohol content, the presence or absence of malt and hops, the presence or absence of fermentation, and the presence or absence of sparkling.

The container filled with the beer taste beverage is not particularly limited. Specific examples thereof include glass bottles, cans, barrels, and flexible containers. Examples of the flexible container include containers obtained by molding flexible resins such as polyethylene (PE), polypropylene (PP), an ethylene/vinyl alcohol copolymer (EVOH), and polyethylene terephthalate (PET). The flexible container may be formed of a single-layer resin or a multi-layer resin.

Examples of the beverage produced by fermentation with yeasts, such as beer, include non-heat-treated beers (so-called "draft beer") that are not heat-sterilized after being packaged in a container and contain live yeasts used for fermentation. The evaluation of microorganism contamination using the method for identifying a microbial species of a microorganism according to the present invention is also useful for non-heat-treated beer taste beverages that have not been subjected to a heat sterilization treatment after being packaged in a container. For example, the method for identifying a microbial species of a microorganism according to the present invention is performed using a packaged beer taste beverage that has not been subjected to a heat sterilization treatment after being packaged in a container as a test sample. In a case where the test sample does not contain a contamination-causing microorganism, it is evaluated that the packaged beer taste beverage in the same production lot as in the test sample can be shipped as a non-heat-treated beer taste beverage.

### [Examples]

Hereinafter, the present invention will be described in more detail based on the examples, but the present invention is not limited to the following examples.

### [Example 1]

Samples in which colonies were formed in a plate medium were prepared for each of the isolated strains of 12 kinds of bacteria listed in Table 9, and a DNA library was prepared by the following method. The obtained DNA library was analyzed with a nanopore sequencer.

### [Method A-1]

### (1) DNA extraction

25 µL of "Prepman Ultra reagent" (manufactured by Thermo Fisher Scientific) was injected into a 200 µL tube, a trace amount of microbial cells were collected from one colony for each of the 12 isolated strains with the tip of a sterilized chip, and a suspension of microbial cells of isolated and cultured microorganisms was prepared.

The 200 µL tube was set in a thermal cycler and allowed to react with a program at 98°C for 10 minutes. After completion of the reaction, the tube was subjected to a centrifugal treatment for 1 minute using a tabletop centrifuge, 20 µL of the supernatant was collected in a new tube, 80 µL of sterilized water was added thereto, and the mixture was mixed.

### (2) PCR

A Barcoded primer mix (the concentration of each primer was 10 µM) containing equimolar amounts of forward primer and reverse primer listed in Table 4 (12 kinds of forward primers and 12 kinds of reverse primers), and template DNA obtained from process (1) described above, were prepared for the PCR reaction.

A total of 50 µL of a PCR reaction solution obtained by mixing 25 µL of a "SapphireAmp Fast PCR Master Mix" (manufactured by Takara Bio Inc.), 1 µL of the Barcoded primer mix, 5 µL of the template DNA solution, and 19 µL of sterilized water was prepared in a 200 µL tube. The tube was set in a thermal cycler using the following program: treatment at 94°C for 1 minute for one reaction cycle, followed by 30 repeated cycles consist of 98°C for 5 seconds, then at 55°C for 10 seconds, and then at 72°C for 10 seconds, and finished by maintaining the temperature of reaction solution at 4°C.

### (3) DNA purification

DNA purification was performed using magnetic beads "AMPure XP beads" (manufactured by Beckman Coulter Inc.) each having a surface modified with a carboxyl group.

The PCR product obtained in the process (2) described above was transferred to a new 1.5 mL tube, 30 µL of magnetic beads were added thereto, the mixture was mixed by inversion for 5 minutes, and the magnetic beads were allowed to aggregate in one site of the tube using a magnet stand, and the supernatant was removed. The magnetic beads remaining in the tube were washed twice with 200 µL of a 70% ethanol solution, the supernatant was completely removed, the container lid was opened for 30 seconds to air dry the remaining ethanol solution. Thereafter, the magnetic beads were suspended in 10 µL of tris buffer (10 mM Tris-HCl, pH of 8.0, 50 mM NaCl), allowed to stand at room temperature for 2 minutes, and allowed to aggregate in one site of the tube using a magnetic stand, and the supernatant was collected in a new tube.

### (4) Addition of adapter

As the Adapter Y top, the Adapter Y bottom, and the motor protein listed in Table 8, "RAP" provided in "16S Barcoding Kit (SQK-RAB204)" (manufactured by Oxford Nanopore Technologies Inc.) was used.

2 µL of the purified PCR product of each strain obtained in the process (3) described above, 1 µL of RAP, and tris buffer (10 mM Tris-HCl, pH of 8.0, 50 mM NaCl) were mixed in a 1.5 mL tube such that the total amount of the mixture reached 13 µL, thereby preparing a reaction solution. The reaction solution was allowed to stand at room temperature for 5 minutes. The solution after the standing was used as a DNA library.

### [Method A-2]

A method A-2 was performed in the same manner as that for the method A-1 except that "(2) PCR" was carried out according to the following method.

### (2) PCR

1st PCR was performed using template DNA solution obtained in the process (1) and using a primer set listed in Table 5 or 6 (the concentration of each primer was 10 µM), and 2nd PCR was performed using a Barcoded primer mix (10 µM) containing equimolar amounts forward primer andreverse primer listed in Table 7 (12 kinds of forward primers and 12 kinds of reverse primers).

A total of 50 µL of a PCR reaction solution obtained by mixing 25 µL of a "SapphireAmp Fast PCR Master Mix" (manufactured by Takara Bio Inc.), 0.5 µL of the primer set for 1 st PCR, 1.5 µL of the primer set for 2st PCR, 5 µL of the template DNA solution, and 18 µL of sterilized water was prepared in a 200 µL tube. The tube was set in a thermal cycler and treated at 94°C for 1 minute for one reaction cycle, followed by treating the solution for 10 repeated cycles consist of 98°C for 5 seconds, then at 56°C for 10 seconds, and then at 72°C for 10 seconds, then followed by30 repeated cycles consist of 98°C for 5 seconds, then at 62°C for 10 seconds, and then at 72°C for 10 seconds, and finished by maintaining the temperature of the reaction solution at 4°C.

### [Method B]

### (1) DNA extraction

DNA extraction from microbial cells was performed using a "MagAttract HMW DNA Kit" (manufactured by QIAGEN N. V.).

First, each of 12 isolated strains was cultured at approximately 10⁹ cells/mL, collected in a 1.5 mL tube such that the total amount thereof reached 1 mL, and centrifuged for 5 minutes using a tabletop centrifuge to remove the supernatant, thereby collecting the microbial cells. 160 µL of Buffer P1 (manufactured by QIAGEN N. V.) was added to completely resuspend the collected microbial cells, then0 µL of lysozyme (100 mg/mL) was added thereto, and the mixture was mixed well by tapping. Next, the tube was set in a thermomixer and incubated at 37°C and 900 rpm for 30 minutes. After the incubation, 20 µL of proteinase K was added thereto and mixed well by tapping, and the tube was set in a thermomixer and incubated at 56°C and 900 rpm for 30 minutes. Thereafter, 4 µL of RNase (100 mg/mL) was added thereto, and the mixture was allowed to react at room temperature for 2 minutes.

Next, 150 µL of Buffer AL (manufactured by QIAGEN N. V.) was added to the tube and mixed well, and 15 µL of MagAttract Suspension G (manufactured by QIAGEN N. V.) and 280 µL of Buffer MB (manufactured by QIAGEN N. V.) were added, and then mixed well. Thereafter, the tube was allowed to react at room temperature and 1400 rpm for 3 minutes and set in Magnetic Rack (manufactured by QIAGEN N. V) to aggregate the residue, and the supernatant was completely removed. Next, 700 µL of Buffer MW1 (manufactured by QIAGEN N. V.) was added to the tube and allowed to react at room temperature and 1400 rpm for 1 minute, then the tube was set in Magnetic Rack to aggregate the residue, and the supernatant was completely removed.

Next, 700 µL of Buffer PE (manufactured by QIAGEN N. V.) was added to the residue in the tube and allowed to react at room temperature and 1400 rpm for 1 minute, then the tube was set in Magnetic Rack to aggregate the residue, and the supernatant was completely removed. Next, 700 µL of Buffer MW1 (manufactured by QIAGEN N. V.) was added to the tube and allowed to react at room temperature and 1400 rpm for 3 minutes, then the tube was set in Magnetic Rack to aggregate the residue, and the supernatant was completely removed. In a state where the tube was set in the Magnetic Rack, 700 µL of sterilized water was added thereto, such that the sterilized water was not directly poured onto the residue, and the mixture was allowed to react at room temperature for 1 minute, the tube was set in the Magnetic Rack, and the supernatant was completely removed.

The tube was taken out from the Magnetic Rack, 100 µL of Buffer AE (manufactured by QIAGEN N. V.) was added thereto, and the tube was set in a thermomixer and allowed to react at 1400 rpm for 3 minutes. Thereafter, the tube was set in the Magnetic Rack, allowed to stand for 1 minute, then100 µL of the supernatant was transferred to a new 1.5 mL tube after 1 minute.

### (2) PCR

A Barcoded primer mix (the concentration of each primer was 10 µM) containing equimolar amounts forward primer and reverse primer listed in Table 4(12 kinds of forward primers and 12 kinds of reverse primers), and template DNA solution obtained in the process (1) described above were prepared for the PCR reaction.

A total of 50 µL of a PCR reaction solution obtained by mixing 25 µL of a "Long Amp Taq 2X master mix" (manufactured by New England Biolabs), 1 µL of the Barcoded primer mix, 5 µL of the template DNA solution, and 19 µL of sterilized water was prepared in a 200 µL tube. The tube was set in a thermal cycler and treated at 95°C for 1 minute for one reaction cycle, followed by 25 repeated cycles consist of 95°C for 20 seconds, then at 55°C for 30 seconds, and then at 65°C for 2 seconds, then followed by 65°C for 5 minutes and then at 4°C.

### (3) DNA purification

The DNA purification was performed on the PCR product obtained in the process (2) described above in the same manner as in the method A-1.

### (4) Addition of adapter

The amount of DNA of the purified PCR product obtained in the process (3) described above was quantified by a spectrometric absorbance-measuring method using a nucleic acid quantification device "Qubit Fluorometer" (manufactured by Thermo Fisher Scientific).

Two 1.5 mL tubes were prepared, and equal amounts of PCR products prepared from 6 kinds of microbial strains among 12 kinds of microbial strains were added to one of the tubes such that the total amount thereof reached 50 to 100 fmol, and equal amounts of PCR products prepared from the remaining 6 kinds of microbial strains were added to the remaining one tube such that the total amount thereof reached 50 to 100 fmol. A reaction solution was prepared by mixing 1 µL of RAP and tris buffer (10 mM Tris-HCl, pH of 8.0, 50 mM NaCl) in each of the two 1.5 mL tubes such that the total amount thereof reached 13 µL. The reaction solution was allowed to stand at room temperature for 5 minutes. The solution after the standing was used as a DNA library.

The time required from the DNA extraction to the completion of the preparation of the DNA library was approximately 90 minutes in a case of using the method A-1, 100 minutes in a case of using the method A-2, and 260 minutes in a case of using the method B.

The DNA concentration of the template DNA solution (the extraction liquid after DNA extraction) and the DNA concentration of the finally prepared DNA library were quantified according to the spectrometric absorbance-measuring method using "Qubit Fluorometer" (manufactured by Thermo Fisher Scientific) for each of the methods.

**[Table 9]**

| | | Template DNA solution [ng/µL] | | DNA library [ng/uL] | | |
|---|---|---|---|---|---|---|
| | Microbial species | A-1, A-2 | B | B | A-1 | A-2 |
| 1 | Lactobacillus brevis | 0.088 | 59.8 | 5 | 2.89 | 5.11 |
| 2 | Lactobacillus lindneri | ≦0,01 | 18.1 | 22 | 5.11 | 6.61 |
| 3 | Lactobacillus paracollinoides | 0.185 | 3.54 | 1.13 | 2.13 | 1.99 |
| 4 | Pediococcus damnosus | 0.272 | Not measured | Not measured | 5.55 | 5.04 |
| 5 | Pectinatus cerevisiiphilus | 0.078 | 17.9 | Outside the range (low) | 7.26 | 4.9 |
| 6 | Pectinatus frisingensis | 0.653 | 30.8 | 0.656 | 6.01 | 2.68 |
| 7 | Megasphaera cerevisiae | 0.188 | 14.7 | 0.11 | 3.05 | 6.02 |
| 8 | Lactobacillus coryniformis | 0.333 | 9.28 | 17.6 | 15.7 | 3.77 |
| 9 | Pediococcus claussenii | 0.084 | 0.414 | 1.67 | 1.78 | 1.92 |
| 10 | Lactobacillus plantarum | 0.09 | 18.4 | 8.86 | 8.52 | 1.28 |
| 11 | Lactobacillus paracasei | 0.33 | 1.7 | 4.34 | 4.49 | 3.82 |
| 12 | Cutibacterium acnes | 0.393 | 8.66 | 0.418 | 0.331 | 2.02 |

As a result, in the DNA extraction methods of the methods A-1 and A-2, the amount of collected DNA was clearly smaller than that of the method B, and the amount of template DNA in the reaction solution of PCR was only approximately 0.5 ng. However, PCR products were obtained without any problems, and the time required for the reaction was reduced in the case of the methods A-1 and A-2 as compared with the method B of the related art, and a DNA library containing a sufficient amount of DNA to be analyzed with a nanopore sequencer was able to be prepared.

### [Analysis using nanopore sequencer]

The prepared DNA library was loaded into the Flow cell of the nanopore sequencer "MinION" (manufactured by Oxford Nanopore Technologies Inc.), and the base sequence of the DNA was determined based on the change in current generated in a case where one DNA molecule was allowed to pass through the nanopore, thereby obtaining base sequence output data. The determined base sequence of each DNA molecule is referred to as a "read". The microbial species was identified for each read by referring to the database "EPI2ME" (manufactured by Oxford Nanopore Technologies Inc.). The analysis results of the DNA libraries prepared by each method are listed in Tables 10 and 11. Table 10 shows the microbial species identified in the top two in a descending order of the number of reads. The number in parentheses is the number of reads identified as each microbial species. Table 11 shows the hit ratio and average accuracy of each analysis result. The hit ratio is the ratio (%) of the number of reads that were able to identify the correct microbial species to the total number of reads of the microbial species identified in the top two in a descending order of the number of reads. The average accuracy is an average value of the homology (%) of the correct base sequence and each read.

**[Table 10]**

| | Microbial sample | Method | Identified microbial species |
|---|---|---|---|
| 1 | Lactobacillus brevis | B | Lactobacillus brevis (16386) Lactobacillus hammesii (164) |
| | | A-1 | Lactobacillus brevis (378) Lactobacillus hammesii (74) |
| | | A-2 | Lactobacillus brevis (2357) Lactobacillus hammesii (785) |
| 2 | Lactobacillus lindneri | B | Lactobacillus lindneri (15752) Lactobacillus sanfrancisnensis (1072) |
| | | A-1 | Lactobacillus lindneri (346) Lactobacillus sanfrancisnensis (23) |
| | | A-2 | Lactobacillus lindneri (11562) Lactobacillus sanfrancisnensis (249) |
| 3 | Lactobacillus paracollinoides | B | Lactobacillus paracollinoides (14487) Lactobacillus collinoides (848) |
| | | A-1 | Lactobacillus paracollinoides (4816) Lactobacillus pentosus (288) |
| | | A-2 | Lactobacillus paracollinoides (719) Lactobacillus collinoides (31) |
| 4 | Pediococcus damnosus | B | N/A |
| | | A-1 | Pediococcus damnosus (529) Pediococcus inopinatus (63) |
| | | A-2 | Pediococcus damnosus (309) Pediococcus inopinatus (39) |
| 5 | Pectinatus cerevisiiphilus | B | Pectinatus cerevisiiphilus (18047) Pectinatus haikarae (33) |
| | | A-1 | Pectinatus cerevisiiphilus (751) Megasphaera cerevisiae (1) |
| | | A-2 | Pectinatus cerevisiiphilus (2437) Pectinatus haikarae (9) |
| 6 | Pectinatus frisingensis | B | Pectinatus frisingensis (16950) Pectinatus portalensis (191) |
| | | A-1 | Pectinatus frisingensis (339) Megasphaera cerevisiae (1) |
| | | A-2 | Pectinatus frisingensis (1430) Pectinatus portalensis (20) |
| 7 | Megasphaera cerevisiae | B | Megasphaera cerevisiae (11448) Megasphaera paucivorans (645) |
| | | A-1 | Megasphaera cerevisiae (619) Megasphaera paucivorans (34) |
| | | A-2 | Megasphaera cerevisiae (2716) Megasphaera paucivorans (223) |
| 8 | Lactobacillus cornyformis | B | Lactobacillus comyformis (19379) Lactobacillus manihotivorans (384) |
| | | A-1 | Lactobacillus comyformis (16542) Lactobacillus manihotivorans (311) |
| | | A-2 | Lactobacillus cornyformis (9833) Lactobacillus manihotivorans (170) |
| 9 | Pediococcus claussenii | B | Pediococcus claussenii (13815) Pediococcus pentosaceus (855) |
| | | A-1 | Pediococcus claussenii (8742) Pediococcus pentosaceus (487) |
| | | A-2 | Pediococcus claussenii (3270) Pediococcus pentosaceus (215) |
| 10 | Lactobacillus plantarum | B | Lactobacillus plantarum (5367) Lactobacillus pentosus (5935) |
| | | A-1 | Lactobacillus plantarum (3672) Lactobacillus pentosus (3987) |
| | | A-2 | Lactobacillus plantarum (949) Lactobacillus pentosus (1346) |
| 11 | Lactobacillus paracasei | B | Lactobacillus paracasei (13105) Lactobacillus casei (267) |
| | | A-1 | Lactobacillus paracasei (8922) Lactobacillus casei (190) |
| | | A-2 | Lactobacillus paracasei (6566) Lactobacillus zeae (210) |
| 12 | Cutibacterium acnes | B | Cutibacterium acnes (5359) Propionibacterium cyclohexanicum(17) |
| | | A-1 | N/A |
| | | A-2 | Cutibacterium acnes (6850) Lactobacillus cornyformis(27) |

**[Table 11]**

| | | Hit Ratio (%) | | | Average accuracy (single read) | | |
|---|---|---|---|---|---|---|---|
| | Microbial species | Method B | Method A-1 | Method A-2 | Method B | Method A-1 | Method A-2 |
| 1 | Lactobacillus brevis | 99.0 | 83.6 | 75.0 | 89.9 | 88.5 | 90.4 |
| 2 | Lactobacillus lindneri | 93.6 | 93.8 | 97.9 | 90.1 | 89.1 | 87.6 |
| 3 | Lactobacillus paracollinoides | 94.5 | 94.4 | 95.9 | 90.3 | 90.0 | 90.9 |
| 4 | Pediococcus damnosus | N/A | 89.4 | 88.8 | N/A | 89.4 | 91.3 |
| 5 | Pectinatus cerevisiiphilus | 99.8 | 99.9 | 99.6 | 88.6 | 88.2 | 90.3 |
| 6 | Pectinatus frisingensis | 98.9 | 99.7 | 98.6 | 89 | 88.7 | 90.8 |
| 7 | Megasphaera cerevisiae | 94.7 | 94.8 | 92.4 | 87.8 | 87.7 | 89.7 |
| 8 | Lactobacillus cornyformis | 98.1 | 98.2 | 98.3 | 89.2 | 89.7 | 88.6 |
| 9 | Pediococcus claussenii | 94.2 | 94.7 | 93.8 | 89.3 | 89.9 | 89.2 |
| 10 | Lactobacillus plantarum | 47.5 | 47.9 | 41.4 | 89.4 | 90.1 | 89.4 |
| 11 | Lactobacillus paracasei | 98.0 | 97.9 | 96.9 | 89.4 | 89.9 | 89.1 |
| 12 | Cutibacterium acnes | 99.7 | N/A | 99.6 | 88.3 | N/A | 88.6 |
| | Average | 92.5 | 90.4 | 89.9 | 89.2 | 89.2 | 89.7 |

The hit ratio and the average accuracy of each DNA library prepared by the methods A-1 and A-2 were almost the same as those of the DNA library prepared by the method B of the related art.

Further, the hit ratio and the average accuracy (single read) of Lactobacillus brevis (No. 1) in a case where the method A-1 or the method A-2 was used were poor as compared with a case where the method B was used. The reason for this is considered to be that the base sequences of 16S rDNA of Lactobacillus brevis and Lactobacillus hammesii were extremely similar, and thus the base sequences were not able to be distinguished from each other by PCR carried out this time. The ratio of the total read that identified Lactobacillus brevis and the read that identified Lactobacillus hammesii to the total number of reads was approximately 100% in both methods.

Further, the hit ratio of Lactobacillus plantarum (No. 10) was less than 50% in both methods, which was low, and the reason for this is considered to be that the base sequences of 16S rDNA of Lactobacillus plantarum and Lactobacillus pentosus were extremely similar, and thus the base sequences were not able to be distinguished from each other by PCR carried out this time. The ratio of the read that identified Lactobacillus plantarum and the read that identified Lactobacillus pentosus to the total number of reads was approximately 100% in both methods.

As shown in the results, it was confirmed that even in a case where the preparation methods A-1 and A-2 are used, the microbial species can be identified by the nanopore sequencer with the same accuracy as in the related art.

Further, the consensus base sequence was determined for each bacterium from the obtained base sequence output data. The microbial species was identified based on the consensus base sequence using a basic local alignment search tool (BLAST). The results are listed in Table 12. As a result, the homology in all the 12 kinds of bacteria was 98.4% or greater which was extremely high. As shown in the results, it was confirmed that the identification accuracy was able to be improved depending on the microbial species by using the consensus base sequence from the base sequence output data obtained from nanopore sequencer to identify the microbial species.

**[Table 12]**

| No | Microorganism | Identified microbial species | Homology | Note |
|---|---|---|---|---|
| 1 | Lactobacillus brevis | Lactobacillus brevis | 99.4% | |
| 2 | Lactobacillus coryniformis | Lactobacillus coryniformis | 98.8% | |
| 3 | Lactobacillus lindneri | Lactobacillus lindneri | 99.2% | |
| 4 | Lactobacillus plantarum | Lactobacillus plantarum | 99.6% | Unable to distinguish |
| | | Lactobacillus pentosus | 99.6% | |
| 5 | Pediococcus claussenii | Pediococcus claussenii | 99.4% | |
| 6 | Lactobacillus paracasei | Lactobacillus paracasei | 98.8% | |
| 7 | Lactobacillus paracollinoides | Lactobacillus paracollinoides | 99.3% | |
| 8 | Pediococcus damnosus | Pediococcus damnosus | 98.6% | |
| 9 | Megasphaera cerevisiae | Megasphaera cerevisiae | 98.4% | |
| 10 | Pectinatus cerevisiiphilus | Pectinatus cerevisiiphilus | 98.8% | |
| 11 | Pectinatus frisingensis | Pectinatus frisingensis | 98.8% | |
| 12 | Cutibacterium acnes | Cutibacterium acnes | 98.4% | |

### [Example 2]

Samples in which colonies were formed in a plate medium were prepared for each of isolated strains of three kinds of fungi (Dekkera bruxellensis, Dekkera anomala, and Zygosaccharomyces sp. (bisporus/parabailli)) to prepare a DNA library according to the following method. The obtained DNA library was analyzed with a nanopore sequencer.

### [Method A-3]

### (1) DNA extraction

DNA extraction from the microbial cells was performed in the same manner as that for the method A-1.

### (2) PCR

Template DNA solution obtained in the process (1) described above, and a primer mix of the Es1-forward primer and the Es 1-reverse primer listed in Table 2 (the concentration of each primer was 10 µM) were used

A total of 50 µL of a PCR reaction solution obtained by mixing 25 µL of a "SapphireAmp Fast PCR Master Mix" (manufactured by Takara Bio Inc.), 0.4 µL of the primer mix, 5 µL of the template DNA solution, and 19.6 µL of sterilized water was prepared in a 200 µL tube. The tube was set in a thermal cycler and treated at 94°C for 1 minute for one reaction cycle, followed by treating the solution for 30 repeated cycles consist of 98°C for 5 seconds, then at 57°C for 5 seconds, and then at 72°C for 10 seconds, and finished by maintaining the temperature of the reaction solution at 4°C.

### (3) Addition of adapter

The repair and the polyadenylation of the end of the PCR product obtained in the process (2) described above was performed using "NEBNext FFPE DNA Repair Mix" (manufactured by New England Biolabs) and "Ultra II End-prep enzyme mix" (manufactured by New England Biolabs).

A total of 60 µL of a reaction solution obtained by mixing 25 µL of the PCR product obtained in the process (2) described above, 1 µL of DNA CS (DNA calibration strand: 3.6 kb amplicon of the λ genome), 3.5 µL of NEB Next FFPE DNA Repair Buffer (manufactured by New England Biolabs), 2 µL of NEB Next FFPE DNA Repair Mix (manufactured by New England Biolabs), 3.5 µL of Ultra II End-prep reaction buffer (manufactured by New England Biolabs), and 3 µL of Ultra II End-prep enzyme mix (manufactured by New England Biolabs) was prepared in a 200 µL tube. The tube was set in a thermal cycler and allowed to react with a program at 20°C for 5 minutes and then at 65°C for 5 minutes.

Next, an adapter was added to the PCR product whose end had been repaired. As the Adapter Y top, the Adapter Y bottom, and the motor protein listed in Table 8, the "Adapter Mix (AMX)" provided in the "Ligation Sequencing Kit (SQK-LSK109)" (manufactured by Oxford Nanopore Technologies Inc.) was used.

60 µL of the reaction solution after the repair reaction, 25 µL of Ligation Buffer (provided in the "Ligation Sequencing Kit (SQK-LSK109)" (manufactured by Oxford Nanopore Technologies Inc.)), 10 µL of NEB Next Quick T4 DNA Ligase (manufactured by New England Biolabs), and 5 µL of AMX were mixed such that the total amount thereof reached 100 µL, thereby preparing a reaction solution. The reaction solution was allowed to stand at room temperature for 10 minutes for reaction.

40 µL of magnetic beads "AMPure XP beads" (manufactured by Beckman Coulter Inc.) were added to the solution after the standing, the solution was mixed by inversion for 5 minutes, and the magnetic beads were allowed to aggregate in one site of the tube using a magnet stand, and the supernatant was removed. The magnetic beads remaining in the tube were washed once with S Fragment Buffer (SFB) (manufactured by Beckman Coulter Inc.). the supernatant was completely removed, then the container lid was opened for 30 seconds to air dry the remaining solution. Thereafter, the magnetic beads were suspended in 15 µL of Elution Buffer (EB) (manufactured by Beckman Coulter Inc.), allowed to stand at room temperature for 10 minutes, and allowed to aggregate in one site of the tube using a magnetic stand, and the supernatant was collected in a new tube. The collected supernatant was used as a DNA library.

### (5) Analysis using nanopore sequencer

The prepared DNA library was loaded into the Flow cell of the nanopore sequencer "MinION" in the same manner as in Example 1, and the consensus base sequence was determined from the obtained base sequence output data. The microbial species was identified based on the consensus base sequence using BLAST.

**[Table 13]**

| Microorganism | Identified microbial species | Homology |
|---|---|---|
| Dekkera bruxellensis | Dekkera bruxellensis | 97% |
| Dekkera anomala | Dekkera anomala | 98% |
| Zygosaccharomyces sp. (bisporus/parabailli) | Zygosaccharomyces bisporus/ Zygosaccharomyces parabailli | 97% / 89% |

Table 13 shows the analysis results of the DNA library prepared from the microbial cells of each microorganism using the nanopore sequencer. As a result, the correct microbial species was able to be identified with high accuracy for all three kinds of fungi using the nanopore sequencer. Further, in a case where "EPI2ME" was used as the database to be referenced, none of the fungi were able to be identified. Further, in a case where the identification was performed using BLAST based on the base sequence output data instead of the consensus base sequence, none of the fungi were able to be identified.

### [Example 3]

Samples in which colonies were formed in a plate medium were prepared for each of the isolated strains of 31 kinds of bacteria listed in Tables 14 and 15 and 29 kinds of fungi listed in Table 16, and a DNA library was prepared by the following method. The obtained DNA library was analyzed with a nanopore sequencer.

### [Method C]

### (1) DNA extraction

DNA extraction from the microbial cells was performed in the same manner as that for the method A-1.

### (2) PCR

Template DNA solution obtained in the process (1) described above , and a primer mix of the Pm1-forward primer and the Pm1-reverse primer listed in Table 4 (the concentration of each primer was 10 µM) was used for bacteria. A primer mix of the Em1-forward primer and the Em3-reverse primer listed in Table 4 (the concentration of each primer was 10 µM) was used for fungi.

A total of 25 µL of a PCR reaction solution obtained by mixing 12.5 µL of a "Q5 High-Fidelity DNA polymerase" (manufactured by New England Biolabs), 0.2 µL of the primer mix, 2.5 µL of the template DNA solution, and 9.8 µL of sterilized water was prepared in a 200 µL tube. The tube was set in a thermal cycler and treated at 98°C for 30 seconds for one reaction cycle, followed by treating the solution for 30 repeated cycles consist of 98°C for 5 seconds, then at 55°C for 10 seconds, and then at 72°C for 30 seconds s, and finished by maintaining the temperature of the reaction solution at 4°C.

### (3) Addition of adapter

The repair and the polyadenylation of the end of the PCR product obtained in the process (2) described above was performed using "NEBNext FFPE DNA Repair Mix" (manufactured by New England Biolabs) and "Ultra II End-prep enzyme mix" (manufactured by New England Biolabs).

A total of 30 µL of a reaction solution obtained by mixing 12.5 µL of a mixture of the PCR product in the process (2) described above with a maximum of six barcodes, 0.5 µL of DNA CS (DNA calibration strand: 3.6 kb amplicon of the λ genome), 1.75 µL of NEB Next FFPE DNA Repair Buffer (manufactured by New England Biolabs), 1 µL of NEB Next FFPE DNA Repair Mix (manufactured by New England Biolabs), 1.75 µL of Ultra II End-prep reaction buffer (manufactured by New England Biolabs), and 1.5 µL of Ultra II End-prep enzyme mix (manufactured by New England Biolabs) was prepared in a 200 µL tube. The tube was set in a thermal cycler and allowed to react with a program at 20°C for 5 minutes and then at 65°C for 5 minutes.

Next, an adapter was added to the PCR product whose end had been repaired. As the Adapter Y top, the Adapter Y bottom, and the motor protein listed in Table 8, the "Adapter Mix (AMX)" provided in the "Ligation Sequencing Kit (SQK-LSK109)" (manufactured by Oxford Nanopore Technologies Inc.) was used.

30 µL of the reaction solution after the repair reaction, 13 µL of Ligation Buffer (provided in the "Ligation Sequencing Kit (SQK-LSK109)" (manufactured by Oxford Nanopore Technologies Inc.)), 5 µL of NEB Next Quick T4 DNA Ligase (manufactured by New England Biolabs), and 2 µL of AMX were mixed such that the total amount thereof reached 50 µL, thereby preparing a reaction solution. The reaction solution was allowed to stand at room temperature for 10 minutes for reaction.

40 µL of magnetic beads "AMPure XP beads" (manufactured by Beckman Coulter Inc.) were added to the solution after the standing, the solution was mixed by inversion for 5 minutes, and the magnetic beads were allowed to aggregate in one site of the tube using a magnet stand, and the supernatant was removed. The magnetic beads remaining in the tube were washed once with S Fragment Buffer (SFB) (manufactured by Beckman Coulter Inc.). the supernatant was completely removed, then the container lid was opened for 30 seconds to air dry the remaining. Thereafter, the magnetic beads were suspended in 10 µL of Elution Buffer (EB) (manufactured by Beckman Couller Inc.), allowed to stand at room temperature for 10 minutes, and allowed to aggregate in one site of the tube using a magnetic stand, and the supernatant was collected in a new tube. The collected supernatant was used as a DNA library.

### (5) Analysis using nanopore sequencer

The prepared DNA library was loaded into the Flow cell of the nanopore sequencer "MinION" in the same manner as in Examples 1 and 2, and the consensus base sequence was determined from the obtained base sequence output data. The microorganism species was identified by calculating the hit ratio and the homology using "EPI2ME" and BLAST based on the consensus base sequence or the base sequence output data.

**[Table 14]**

| No | Microorganism | Identified microbial species | Number of reads | Hit ratio (%) |
|---|---|---|---|---|
| 1 | Lactobacillus acetotolerans | Lactobacillus acetotolerans | 1948 | 97.4 |
| | | Lactobacillus intestinalis | 53 | |
| 2 | Lactobacillus backii | Lactobacillus backii | 1767 | 90.8 |
| | | Lactobacillus iwatensis | 180 | |
| 3 | Lactobacillus brevis | Lactobacillus brevis | 1850 | 83.2 |
| | | Lactobacillus hammesii | 374 | |
| 4 | Lactobacillus buchneri | Lactobacillus buchneri | 4221 | 91.8 |
| | | Lactobacillus parabuchneri | 376 | |
| 5 | Lactobacillus casei | Lactobacillus casei | 2497 | 82.5 |
| | | Lactobacillus rhamnosus | 528 | |
| 6 | Lactobacillus coryniformis | Lactobacillus coryniformis | 1513 | 98.8 |
| | | Lactobacillus plantarum | 19 | |
| 7 | Lactobacillus curtus | Lactobacillus curtus | 1738 | 79.1 |
| | | Lactobacillus siliginis | 459 | |
| 8 | Lactobacillus hammesii | Lactobacillus hammesii | 2963 | 95.7 |
| | | Lactobacillus parabrevis | 132 | |
| 9 | Lactobacillus harbinensis | Lactobacillus harbinensis | 4518 | 99.1 |
| | | Lactobacillus perolens | 39 | |
| 10 | Lactobacillus lindneri | Lactobacillus lindneri | 1309 | 95.3 |
| | | Lactobacillus florum | 64 | |
| 11 | Lactobacillus malefermentans | Lactobacillus malefermentans | 1391 | 99.3 |
| | | Lactobacillus oryzae | 10 | |
| 12 | Lactobacillus nagelii | Lactobacillus nagelii | 1802 | 98.5 |
| | | Lactobacillus ghanensis | 27 | |
| 13 | Lactobacillus parabuchneri | Lactobacillus parabuchneri | 1233 | 96.0 |
| | | Lactobacillus fructivorans | 52 | |
| 14 | Lactobacillus paracasei | Lactobacillus paracasei | 3702 | 92.6 |
| | | Lactobacillus rhamnosus | 298 | |
| 15 | Lactobacillus paracollinoides | Lactobacillus paracollinoides | 3597 | 96.2 |
| | | Lactobacillus collinoides | 143 | |
| 16 | Lactobacillus paucivorans | Lactobacillus paucivorans | 1761 | 92.5 |
| | | Lactobacillus parabrevis | 143 | |

**[Table 15]**

| No | Microorganism | Identified microbial species | Number of reads | Hit ratio (%) |
|---|---|---|---|---|
| 17 | Lactobacillus pentosus | Lactobacillus pentosus | 4678 | 72.0 |
| | | Lactobacillus plantarum | 1817 | |
| 18 | Lactobacillus plantarum | Lactobacillus pentosus | 1444 | 51.2 |
| | | Lactobacillus plantarum | 1374 | |
| 19 | Lactobacillus reuteri | Lactobacillus reuteri | 5189 | 98.4 |
| | | Lactobacillus mucosae | 86 | |
| 20 | Lactobacillus rhamnosus | Lactobacillus rhamnosus | 1197 | 91.9 |
| | | Lactobacillus paracasei | 106 | |
| 21 | Lactobacillus rossiae | Lactobacillus rossiae | 4530 | 98.2 |
| | | Lactobacillus siliginis | 85 | |
| 22 | Lactobacillus siliginis | Lactobacillus siliginis | 3503 | 95.6 |
| | | Lactobacillus rossiae | 160 | |
| 23 | Pediococcus claussenii | Pediococcus claussenii | 1238 | 94.3 |
| | | Pediococcus pentosaceus | 75 | |
| 24 | Pediococcus damnosus | Pediococcus damnosus | 1978 | 89.3 |
| | | Pediococcus inopinatus | 236 | |
| 25 | Pediococcus inopinatus | Pediococcus inopinatus | 24569 | 96.1 |
| | | Pediococcus cellicola | 996 | |
| 26 | Megasphaera cerevisiae | Megasphaera cerevisiae | 5745 | 91.8 |
| | | Megasphaera paucivorans | 516 | |
| 27 | Megasphaera paucivorans | Megasphaera paucivorans | 2033 | 95.7 |
| | | Megasphaera sueciensis | 91 | |
| 28 | Megasphaera sueciensis | Megasphaera sueciensis | 1189 | 95.0 |
| | | Megasphaera paucivorans | 63 | |
| 29 | Pectinatus cerevisiiphilus | Pectinatus cerevisiphilus | 3873 | 99.2 |
| | | Pectinatus haikarae | 30 | |
| 30 | Pectinatus frisingensis | Pectinatus frisingensis | 1268 | 98.2 |
| | | Pectinatus portalensis | 23 | |
| 31 | Pectinatus haikarae | Pectinatus haikarae | 1688 | 99.5 |
| | | Pectinatus cerevisiphilus | 9 | |

**[Table 16]**

| No. | Microorganism | Identified microbial species | Homology |
|---|---|---|---|
| 1 | Brettanomyces bruxellensis | Brettanomyces bruxellensis | 99.0 |
| 2 | Brettanomyces custorisinamus | Brettanomyces custorisinamus | 98.8 |
| 3 | Brettanomyces naardenensis | Brettanomyces naardenensis | 99.1 |
| 4 | Brettanomyces nanus | Brettanomyces nanus | 99.8 |
| 5 | Candida boidinii | Candida boidinii | 99.0 |
| 6 | Candida intermedia | Candida intermedia | 98.8 |
| 7 | Candida krusei | Pichia kudriavzevii | 99.9 |
| 8 | Candida tropicalis | Candida tropicalis | 99.9 |
| 9 | Cryptococcus curvatus | Cutaneotrichosporon curvatus | 99.7 |
| 10 | Cryptococcus liquefaciens | Naganishia liquefaciens | 97.4 |
| 11 | Dekkera anomala | Dekkera anomala | 98.2 |
| 12 | Hanseniaspora uvarum | Hanseniaspora uvarum | 99.9 |
| 13 | Hanseniaspora vineae | Hanseniaspora vineae | 99.4 |
| 14 | Issatchenkia occidentalis | Pichia occidentalis | 100.0 |
| 15 | Lanchancea kluyeri | Lanchancea kluyveri | 99.9 |
| 16 | Ogataea minuta var. nonfermentans | Ogataea nonfermentans | 99.9 |
| 17 | Pichia guiliermondii | Pichia guiliermondii | 99.8 |
| 18 | Rhodotorula mucilaginosa | Rhodotorula mucilaginosa | 99.8 |
| 19 | Saccharomyces bayanus | Saccharomyces eubayanus | 99.9 |
| 20 | Saccharomyces cerevisiae | Saccharomyces cerevisiae | 97.3 |
| 21 | Saccharomyces cerevisiae var. diastaticus | Saccharomyces cerevisiae | 98.6 |
| 22 | Saccharomyces pastorianus | Saccharomyces cerevisiae | 90.0 |
| 23 | Torulaspora delbrueckii | Torulaspora delbrueckii | 99.9 |
| 24 | Torulaspora microellipsoides | Torulaspora microellipsoides | 99.6 |
| 25 | Wickerhamomyces anomalus | Wickerhamomyces anomalus | 100.0 |
| 26 | Zyosaccharomyces bailii | Zygosaccharomyces bailii | 98.4 |
| 27 | Zygosaccharomyces fermentati | Zygosaccharomyces fermentati | 100.0 |
| 28 | Zygosaccharomyces florentinus | Zygotoralospora florentina | 99.8 |
| 29 | Zygosaccharomyces rouxii | Zygosaccharomyces rouxii | 100.0 |

Tables 14 to 16 show the analysis results of the DNA library prepared from the microbial cells of each microorganism using the nanopore sequencer. In the columns of "identified microorganisms" in Tables 14 and 15, the upper row shows a microorganism having the highest number of reads, and the lower row shows a microorganism having the second highest number of reads. The hit ratio is the ratio (%) of the number of reads that were able to identify the correct microbial species to the total number of reads of the microbial species identified in the top two in a descending order of the number of reads. As a result, 31 kinds of bacteria and 29 kinds of fungi were able to be identified to the correct microbial species with high accuracy using the nanopore sequencer. However, since some genera Saccharomyces were not distinguishable in the ITS-26S region, Saccharomyces pastorianus (No. 22) was identified as Saccharomyces cerevisiae.

In a case where "EPI2ME" was used as the database to be referenced, at least half of the fungi could not be identified. Further, in a case where the identification was performed using BLAST based on the base sequence output data instead of the consensus base sequence, none of the fungi were able to be identified.

### [Example 4]

Samples in which colonies were formed in a plate medium were prepared for each of the isolated strains of 31 kinds of bacteria and 29 kinds of fungi used in Example 3, and a DNA library was prepared by the following method. The obtained DNA library was analyzed with a nanopore sequencer.

### [Method D]

### (1) DNA extraction

DNA extraction from the microbial cells was performed in the same manner as that for the method A-1.

### (2) PCR

Template DNA solution obtained in the process (1) described above, and a primer mix of the Ps1-forward primer and the Ps1-reverse primer listed in Table 1 (the concentration of each primer was 10 µM) was used for bacteria. A primer mix of the Es1-forward primer and the Es3-reverse primer listed in Table 2 (the concentration of each primer was 10 µM) was used for fungi.

A total of 25 µL of a PCR reaction solution obtained by mixing 12.5 µL of a "Q5 High-Fidelity DNA polymerase" (manufactured by New England Biolabs), 0.2 µL of the primer mix, 2.5 µL of the template DNA solution, and 9.8 µL of sterilized water was prepared in a 200 µL tube. The tube was set in a thermal cycler and treated at 98°C for 30 seconds for one reaction cycle, followed by treating the solution for 30 repeated cycles consist of t 98°C for 5 seconds, then at 55°C for 10 seconds, and then at 72°C for 30 seconds, and finished by maintaining the temperature of the reaction solution at 4°C.

### (3) Fragmentation

A reaction solution obtained by mixing 8 µL of the PCR product obtained in the process (2) described above and 2 µL of any of Fragmentation Mix RBQs 1 to 12 such that the total amount thereof reached 10 µL was prepared in a 200 µL tube. The tube was set in a thermal cycler, treated at 30°C for 1 minute, allowed to react at 80°C for 1 minute, and allowed to react with a program of maintaining the solution at 4°C.

Thereafter, DNA purification was performed in the same manner as that for the method A-1. At this time, a maximum of four samples were mixed in one tube to enable purification.

### (4) Addition of adapter

The DNA quantification of the fragmented product after the purification which were obtained in the process (3) described above were quantified by a spectrometric absorbance-measuring method using a nucleic acid quantifier "Qubit Fluoro meter" (manufactured by Thermo Fisher Scientific).

One 1.5 mL tube was prepared, and equal amounts of the fragmented products after the purification were added to the tube such that the total amount thereof reached 50 to 100 fmol. A reaction solution was prepared by mixing 1 µL of RAP and tris buffer (10 mM Tris-HCl, pH of 8.0, 50 mM NaCl) in this 1.5 mL tube such that the total amount thereof reached 11 µL. The reaction solution was allowed to stand at room temperature for 5 minutes. The solution after the standing was used as a DNA library.

The time required from the DNA extraction to the completion of the preparation of the DNA library was approximately 110 minutes.

### (5) Analysis using nanopore sequencer

The prepared DNA library was loaded into the Flow cell of the nanopore sequencer "MinION" in the same manner as in Examples 1 and 2, and the consensus base sequence was determined from the obtained base sequence output data. The microorganism species was identified by calculating the hit ratio and the homology using "EPI2ME" and BLAST based on the consensus base sequence or the base sequence output data.

**[Table 17]**

| No | Microorganism | Identified microbial species | Number of reads | Hit ratio (%) |
|---|---|---|---|---|
| 1 | Lactobacillus acetotolerans | Lactobacillus acetotolerans | 6160 | 97.8 |
| | | Lactobacillus intestinalis | 136 | |
| 2 | Lactobacillus backii | Lactobacillus backii | 3895 | 88.4 |
| | | Lactobacillus iwatensis | 509 | |
| 3 | Lactobacillus brevis | Lactobacillus brevis | 3263 | 87.4 |
| | | Lactobacillus hammesii | 470 | |
| 4 | Lactobacillus buchneri | Lactobacillus buchneri | 1050 | 83.1 |
| | | Lactobacillus parabuchneri | 214 | |
| 5 | Lactobacillus casei | Lactobacillus casei | 1048 | 90.0 |
| | | Lactobacillus zeae | 116 | |
| 6 | Lactobacillus cornyformis | Lactobacillus cornyformis | 7989 | 99.0 |
| | | Lactobacillus manihotivorans | 78 | |
| 7 | Lactobacillus curtus | Lactobacillus curtus | 2618 | 94.8 |
| | | Lactobacillus rossiae | 145 | |
| 8 | Lactobacillus hammesii | Lactobacillus hammesii | 1814 | 96.9 |
| | | Lactobacillus parabrevis | 58 | |
| 9 | Lactobacillus harbinensis | Lactobacillus harbinensis | 1924 | 98.8 |
| | | Lactobacillus perolens | 23 | |
| 10 | Lactobacillus lindneri | Lactobacillus lindneri | 6577 | 97.1 |
| | | Lactobacillus sanfranciscensis | 199 | |
| 11 | Lactobacillus malefermentans | Lactobacillus malefermentans | 3905 | 99.3 |
| | | Lactobacillus plantarum | 27 | |
| 12 | Lactobacillus nagelii | Lactobacillus nagelii | 4485 | 98.9 |
| | | Lactobacillus ghanensis | 48 | |
| 13 | Lactobacillus parabuchneri | Lactobacillus parabuchneri | 2451 | 95.9 |
| | | Lactobacillus otakiensis | 104 | |
| 14 | Lactobacillus paracasei | Lactobacillus paracasei | 3328 | 92.4 |
| | | Lactobacillus casei | 274 | |
| 15 | Lactobacillus paracolinoides | Lactobacillus paracollinoides | 5460 | 96.3 |
| | | Lactobacillus collinoides | 207 | |
| 16 | Lactobacillus paucivorans | Lactobacillus paucivorans | 3180 | 96.4 |
| | | Lactobacillus parabrevis | 120 | |

**[Table 18]**

| No | Microorganism | Identified microbial species | Number of reads | Hit ratio (%) |
|---|---|---|---|---|
| 17 | Lactobacillus pentosus | Lactobacillus pentosus | 1238 | 56.9 |
| | | Lactobacillus plantarum | 939 | |
| 18 | Lactobacillus plantarum | Lactobacillus plantarum | 2503 | 57.5 |
| | | Lactobacillus pentosus | 1850 | |
| 19 | Lactobacillus reuteri | Lactobacillus reuteri | 2467 | 99.2 |
| | | Lactobacillus frumenti | 21 | |
| 20 | Lactobacillus rhamnosus | Lactobacillus rhamnosus | 1034 | 70.5 |
| | | Lactobacillus paracasei | 433 | |
| 21 | Lactobacillus rossiae | Lactobacillus rossiae | 3916 | 95.4 |
| | | Lactobacillus siliginis | 187 | |
| 22 | Lactobacillus siliginis | Lactobacillus siliginis | 1833 | 94.7 |
| | | Lactobacillus rossiae | 102 | |
| 23 | Pediococcus clausenii | Pediococcus clausenii | 2276 | 97.4 |
| | | Pediococcus pentosaceus | 60 | |
| 24 | Pediococcus damnosus | Pediococcus damnosus | 10039 | 82.7 |
| | | Pediococcus inopinatus | 2097 | |
| 25 | Pediococcus inopinatus | Pediococcus inopinatus | 3343 | 98.9 |
| | | Pediococcus damnosus | 38 | |
| 26 | Megasphaera cerevisae | Megasphaera cerevisiae | 4295 | 94.0 |
| | | Megasphaera massiliensis | 274 | |
| 27 | Megasphaera paucivorans | Megasphaera paucivorans | 3454 | 98.3 |
| | | Megasphaera sueciensis | 61 | |
| 28 | Megasphaera sueciensis | Megasphaera sueciensis | 5758 | 91.2 |
| | | Megasphaera paucivorans | 555 | |
| 29 | Pectinatus cerevisiiphilus | Pectinatus cerevisiiphilus | 8471 | 99.7 |
| | | Pectinatus haikarae | 29 | |
| 30 | Pectinatus frisingensis | Pectinatus frisingensis | 1313 | 96.5 |
| | | Pectinatus portalensis | 48 | |
| 31 | Pectinatus haikarae | Pectinatus haikarae | 3659 | 99.9 |
| | | Pectinatus frisingensis | 4 | |

**[Table 19]**

| No. | Microorganism | Identified microbial species | Homology (%) |
|---|---|---|---|
| 1 | Brettanomyces bruxellensis | Dekkera bmxellensis | 98.0 |
| 2 | Brettanomyces custorisinamus | Brettanomyces custorisinamus | 97.6 |
| 3 | Brettanomyces naardenensis | Brettanomyces naardenensis | 98.4 |
| 4 | Brettanomyces nanus | Brettanomyces nanus | 99.3 |
| 5 | Candida boidinii | Candida boidinii | 97.2 |
| 6 | Candida intermedia | Candida intermedia | 97.0 |
| 7 | Candida krusei | Pichia kudriavzevii | 98.9 |
| 8 | Candida tropicalis | Candida tropicalis | 98.8 |
| 9 | Cryptococcus curvatus | Cutaneotrichosporon curvatus | 99.4 |
| 10 | Cryptococcus liquefaciens | Naganishia liquefaciens | 96.8 |
| 11 | Dekkera anomala | Dekkera anomala | 97.1 |
| 12 | Hanseniaspora uvarum | Hanseniaspora uvarum | 98.9 |
| 13 | Hanseniaspora vineae | Hanseniaspora vineae | 97.3 |
| 14 | Issatchenkia occidentalis | Pichia occidentalis | 97.3 |
| 15 | Lanchancea kluyveri | Lachancea kluyveri | 99.2 |
| 16 | Ogataea minuta var. nonfermentans | Ogataea nonfermentans | 98.4 |
| 17 | Pichia guiliennondii | Pichia guiliermondii | 98.5 |
| 18 | Rhodotorula mucilaginosa | Rhodotorula mucilaginosa | 98.5 |
| 19 | Saccharomyces bayanus | Saccharomyces eubayanaus | 98.6 |
| 20 | Saccharomyces cerevisiae | Saccharomyces cerevisiae | 97.8 |
| 21 | Saccharomyces cerevisiae var. diastaticus | Saccharomyces cerevisiae | 98.0 |
| 22 | Saccharomyces pastorianus | Saccharomyces cerevisiae | 99.2 |
| 23 | Torulaspora delbrueckii | Torulaspora delbrueckii | 99.2 |
| 24 | Torulaspora microellipsoides | Torulaspora microellipsoides | 98.8 |
| 25 | Wickerhamomyces anomalus | Wickerhamomyces anomalus | 98.9 |
| 26 | Zygosaccharomyces bailii | Zygosaccharomyces bailii | 98.2 |
| 27 | Zygosaccharomyces fermentati | Zygosaccharomyces fermentati | 99.3 |
| 28 | Zygosaccharomyces florentinus | Zygotorulaspora florentina | 99.0 |
| 29 | Zygosaccharomyces rouxii | Zygosaccharomyces rouxii | 98.6 |

Tables 17 to 19 show the analysis results of the DNA library prepared from the microbial cells of each microorganism using the nanopore sequencer. In the columns of "identified microorganisms" in Tables 17 and 18, the upper row shows a microorganism having the highest number of reads, and the lower row shows a microorganism having the second highest number of reads. The hit ratio is the same as in Table 14. As a result, 31 kinds of bacteria and 29 kinds of fungi were able to be identified to the correct microbial species with high accuracy using the nanopore sequencer. In a case where "EPI2ME" was used as the database to be referenced, at least half of the fungi could not be identified. Further, in a case where the identification was performed using BLAST based on the base sequence output data instead of the consensus base sequence, none of the fungi were able to be identified.

### [Example 5]

Samples in which colonies were formed in a plate medium were prepared for each of the five kinds of bacteria known as harmful bacteria for beverages and foods, and a microorganism species was identified under the same conditions as in Examples 3 and 4.

**[Table 20]**

| No | Microorganism | Identified microbial species | Number of reads | Hit ratio (%) |
|---|---|---|---|---|
| 1 | Clostridium butyricum | Clostridium butyricum | 2128 | 88.4 |
| | | Clostridium paraputrificum | 280 | |
| 2 | Moorella thermoacetica | Moorella thermoacetica | 405 | 94.6 |
| | | Moorella thermoautotrophica | 23 | |
| 3 | Thermoanaerobacterium thermosaccharolyticum | Thermoanaerobacterium thermosaccharolyticum | 518 | 83.4 |
| | | Thermoanaerobacterium aotearoense | 103 | |
| 4 | Alicyclobacillus acidoterrestris | Alicyclobacillus acidoterrestris | 808 | 99.0 |
| | | Alicyclobacillus hesperidum | 8 | |
| 5 | Alicyclobacillus acidiphilus | Alicyclobacillus acidiphilus | 705 | 90.7 |
| | | Alicyclobacillus hesperidum | 72 | |

**[Table 21]**

| No | Microorganism | Identified microbial species | Number of reads | Hit ratio (%) |
|---|---|---|---|---|
| 1 | Clostridium butyricum | Clostridium butyricum | 2713 | 93.2 |
| | | Clostridium paraputrificum | 199 | |
| 2 | Moorella thermoacetica | Moorella thermoacetica | 708 | 71.4 |
| | | Moorella thermoautotrophica | 283 | |
| 3 | Thermoanaerobacterium thermosaccharolyticum | Thermoanaerobacterium thermosaccharolyticum | 4324 | 99.3 |
| | | Thermoanaerobacterium aotearoense | 31 | |
| 4 | Alicyclobacillus acidoterrestris | Alicyclobacillus acidoterrestris | 4457 | 95.3 |
| | | Alicyclobacillus hesperidum | 218 | |
| 5 | Alicyclobacillus acidiphilus | Alicyclobacillus acidiphilus | 7215 | 84.8 |
| | | Alicyclobacillus hesperidum | 1290 | |

Among the analysis results of the DNA library prepared from the microbial cells of each microorganism using the nanopore sequencer, the results of analysis carried out under the same conditions as in Example 3 are listed in Table 20, and the results of analysis carried out under the same conditions as in Example 4 are listed in Table 21. As a result, the correct microbial species was able to be identified with high accuracy for five kinds of bacteria using the nanopore sequencer.

## Claims

1. A method for preparing a DNA library for identifying a microbial species using a nanopore sequencer, the method comprising:
a DNA extraction step of suspending microbial cells, which have been isolated and cultured, in an alkaline solution having a pH of 8.0 to 8.6, performing a heat treatment on the obtained suspension at 90°C to 100°C for 1 to 15 minutes, and performing a centrifugal treatment on the heat-treated suspension to collect a supernatant;
a PCR step of performing hot start PCR using a polymerase which requires a time of 10 seconds or shorter for 1 kb extension using DNA contained in the supernatant as a template; and
an adapter addition step of adding an adapter, to which a motor protein is bound, to an end of a PCR product obtained in the PCR step.

2. The method for preparing a DNA library according to Claim 1,
wherein the hot start PCR is performed such that one cycle consisting of heat denaturation, annealing, and extension is carried out within 70 seconds.

3. The method for preparing a DNA library according to Claim 1 or 2,
wherein a PCR product of 700 bp or longer is obtained in the hot start PCR.

4. The method for preparing a DNA library according to any one of Claims 1 to 3,
wherein the hot start PCR is performed using:
(1) a set consisting of a forward primer represented by SEQ ID NO: 1 and a reverse primer represented by SEQ ID NO: 2,
(2) a set consisting of a forward primer consisting of a base sequence in which a barcode sequence represented by any of SEQ ID NOs: 3 to 14 is linked to a 3' end of a base sequence represented by SEQ ID NO: 15 and a base sequence represented by SEQ ID NO: 1 is linked to a 3' end of the barcode sequence and a reverse primer consisting of a base sequence in which the barcode sequence is linked to the 3' end of the base sequence represented by SEQ ID NO: 15 and a base sequence represented by SEQ ID NO: 2 is linked to the 3' end of the barcode sequence,
(3) a set consisting of a forward primer represented by SEQ ID NO: 16 and a reverse primer represented by SEQ ID NO: 17, 18, or 19, or
(4) a set, for 1st PCR, consisting of a forward primer consisting of a base sequence in which a base sequence represented by SEQ ID NO: 16 is linked to a 3' end of a base sequence represented by SEQ ID NO: 20 and a reverse primer consisting of a base sequence in which a base sequence represented by SEQ ID NO: 17 is linked to a 3' end of the base sequence represented by SEQ ID NO: 21 and
a set, for 2nd PCR, consisting of a forward primer consisting of a base sequence in which the barcode sequence represented by any of SEQ ID NOs: 3 to 14 is linked to the 3' end of the base sequence represented by SEQ ID NO: 15 and the base sequence represented by SEQ ID NO: 20 is linked to the 3' end of the barcode sequence and a reverse primer consisting of a base sequence in which the barcode sequence represented by any of SEQ ID NOs: 3 to 14 is linked to the 3' end of the base sequence represented by SEQ ID NO: 15 and the base sequence represented by SEQ ID NO: 21 is linked to the 3' end of the barcode sequence.

5. The method for preparing a DNA library according to any one of Claims 1 to 4,
wherein in the adapter addition step, the end of the PCR product is repaired to perform polyadenylation of a 3' end of the PCR product without measurement of the spectrometric absorbance, and a poly(T) adapter is ligated and added to the polyadenylated PCR product.

6. The method for preparing a DNA library according to any one of Claims 1 to 4,
wherein in the adapter addition step, the PCR product is purified using magnetic beads each having a surface modified with a carboxyl group, and an adapter is added to an end of the PCR product without measurement of the spectrometric absorbance of the purified DNA.

7. The method for preparing a DNA library according to any one of Claims 1 to 6,
wherein the microbial cell is a fungus or a bacterium.

8. The method for preparing a DNA library according to any one of Claims 1 to 7,
wherein a process from start of the DNA extraction step to end of the adapter addition step is performed within 200 minutes.

9. A method for identifying a microbial species of a microorganism, comprising:
preparing a DNA library from microbial cells individually isolated and cultured from a test sample using the method for preparing a DNA library according to any one of Claims 1 to 8;
analyzing the DNA library with a nanopore sequencer; and
identifying the microbial species of the microbial cells based on obtained base sequence output data.

10. A method for identifying a microbial species of a microorganism, comprising:
preparing a DNA library from microbial cells individually isolated and cultured from a test sample using the method for preparing a DNA library according to any one of Claims 1 to 8;
analyzing the DNA library with a nanopore sequencer;
determining a consensus base sequence based on obtained base sequence output data; and
identifying a microbial species of the microbial cells based on the determined consensus base sequence.

11. A method for evaluating microorganism contamination in a processed food or drink, the method comprising:
preparing a processed food or drink or a semi-finished product collected in the course of a production process of the processed food or drink as a test sample;
isolating and culturing microorganisms contained in the test sample and identifying a microbial species of each microorganism using the method for identifying a microbial species of a microorganism according to Claim 9 or 10;
evaluating that a semi-finished product and a finished product in the same production lot as in the test sample are contaminated by a contamination-causing microorganism in a case where the test sample contains a contamination-causing microorganism ; and
evaluating that a semi-finished product and a finished product in the same production lot as in the test sample are not contaminated by a contamination-causing microorganism in a case where the test sample does not contain a contamination-causing microorganism.

12. The method for evaluating microorganism contamination according to Claim 11,
wherein the test sample is a processed food or drink before being shipped from a factory,
in a case where the test sample contains a contamination-causing microorganism, it is evaluated that the processed food or drink in the same production lot as in the test sample is not shippable, and
in a case where the test sample does not contain a contamination-causing microorganism, it is evaluated that the processed food or drink in the same production lot as in the test sample is shippable.

13. The method for evaluating microorganism contamination according to Claim 11,
wherein in a case where the test sample contains a contamination-causing microorganism, it is evaluated that the production line from which the test sample has been collected is contaminated with the contamination-causing microorganism, and
in a case where the test sample does not contain a contamination-causing microorganism, it is evaluated that the production line from which the test sample has been collected is not contaminated with the contamination-causing microorganism.

14. The method for evaluating microorganism contamination according to any one of Claims 11 to 13,
wherein the processed food or drink is a packaged beer taste beverage.

15. The method for evaluating microorganism contamination according to Claim 14,
wherein the processed food or drink is a packaged beer taste beverage that has not been subjected to a heat sterilization treatment after being packaged, and
in a case where the test sample does not contain a contamination-causing microorganism, it is evaluated that the packaged beer taste beverage in the same production lot as in the test sample is shippable as a non-heat-treated beer taste beverage.


SEQUENCE LISTING

<110>  Asahi Group Holdings, Ltd.
<120>  Preparation method of DNA library
<130>  PC-29781
<160>  27

<210>  1
<211>  20
<212>  DNA
<213>  Artificial Sequence
<220>
<223>  Description of Artificial Sequence: Synthesized Ps1-forward primer
<400>  1
agagtttgat cmtggctcag                                                    20

<210>  2
<211>  19
<212>  DNA
<213>  Artificial Sequence
<220>
<223>  Description of Artificial Sequence: Synthesized Ps1-reverse primer
<400>  2
ggttaccttg ttacgactt                                                     19

<210>  3
<211>  24
<212>  DNA
<213>  Artificial Sequence
<220>
<223>  Description of Artificial Sequence: Synthesized barcode 16S01
<400>  3
aagaaagttg tcggtgtctt tgtg                                               24

<210>  4
<211>  24
<212>  DNA
<213>  Artificial Sequence
<220>
<223>  Description of Artificial Sequence: Synthesized barcode 16S02
<400>  4
tcgattccgt ttgtagtcgt ctgt                                               24

<210>  5
<211>  24
<212>  DNA
<213>  Artificial Sequence
<220>
<223>  Description of Artificial Sequence: Synthesized barcode 16S03
<400>  5
gagtcttgtg tcccagttac cagg                                               24

<210>  6
<211>  24
<212>  DNA
<213>  Artificial Sequence
<220>
<223>  Description of Artificial Sequence: Synthesized barcode 16S04
<400>  6
ttcggattct atcgtgtttc ccta                                               24

<210>  7
<211>  24

<212>  DNA
<213>  Artificial Sequence
<220>
<223>  Description of Artificial Sequence: Synthesized barcode 16S05
<400>  7
cttgtccagg gtttgtgtaa cctt                                              24

<210>  8
<211>  24
<212>  DNA
<213>  Artificial Sequence
<220>
<223>  Description of Artificial Sequence: Synthesized barcode 16S06
<400>  8
ttctcgcaaa ggcagaaagt agtc                                              24

<210>  9
<211>  24
<212>  DNA
<213>  Artificial Sequence
<220>
<223>  Description of Artificial Sequence: Synthesized barcode 16S07
<400>  9
gtgttaccgt gggaatgaat cctt                                              24

<210>  10
<211>  24
<212>  DNA
<213>  Artificial Sequence
<220>
<223>  Description of Artificial Sequence: Synthesized barcode 16S08
<400>  10
ttcagggaac aaaccaagtt acgt                                              24

<210>  11
<211>  24
<212>  DNA
<213>  Artificial Sequence
<220>
<223>  Description of Artificial Sequence: Synthesized barcode 16S09
<400>  11
aactaggcac agcgagtctt ggtt                                              24

<210>  12
<211>  24
<212>  DNA
<213>  Artificial Sequence
<220>
<223>  Description of Artificial Sequence: Synthesized barcode 16S10
<400>  12
aagcgttgaa acctttgtcc tctc                                              24

<210>  13
<211>  24
<212>  DNA
<213>  Artificial Sequence
<220>
<223>  Description of Artificial Sequence: Synthesized barcode 16S11
<400>  13
gtttcatcta tcggagggaa tgga                                              24

<210>  14
<211>  24

<212>  DNA
<213>  Artificial Sequence
<220>
<223>  Description of Artificial Sequence: Synthesized barcode 16S12
<400>  14
caggtagaaa gaagcagaat cgga                                              24

<210>  15
<211>  15
<212>  DNA
<213>  Artificial Sequence
<220>
<223>  Description of Artificial Sequence: Synthesized common sequence of
primer
<400>  15
atcgcctacc gtgac                                                        15

<210>  16
<211>  18
<212>  DNA
<213>  Artificial Sequence
<220>
<223>  Description of Artificial Sequence: Synthesized Es1-forward primer
<400>  16
tgtacacacc gcccgtcg                                                     18

<210>  17
<211>  22
<212>  DNA
<213>  Artificial Sequence
<220>
<223>  Description of Artificial Sequence: Synthesized Es1-reverse primer
<400>  17
gagctgcatt cccaaacaac tc                                                22

<210>  18
<211>  26
<212>  DNA
<213>  Artificial Sequence
<220>
<223>  Description of Artificial Sequence: Synthesized Es2-reverse primer
<400>  18
aatgcattgg tccgtgtttc aagacg                                            26

<210>  19
<211>  20
<212>  DNA
<213>  Artificial Sequence
<220>
<223>  Description of Artificial Sequence: Synthesized Es3-reverse primer
<400>  19
cttggtccgt gtttcaagac                                                   20

<210>  20
<211>  22
<212>  DNA
<213>  Artificial Sequence
<220>
<223>  Description of Artificial Sequence: Synthesized common sequence of
forward primer
<400>  20
tttctgttgg tgctgatatt gc                                                22

<210>  21
<211>  22
<212>  DNA
<213>  Artificial Sequence
<220>
<223>  Description of Artificial Sequence: Synthesized common sequence of
reverse primer
<400>  21
acttgcctgt cgctctatct tc                                                22

<210>  22
<211>  40
<212>  DNA
<213>  Artificial Sequence
<220>
<223>  Description of Artificial Sequence: Synthesized Em1-forward primer
<400>  22
tttctgttgg tgctgatatt gctgtacaca ccgcccgtcg                             40

<210>  23
<211>  44
<212>  DNA
<213>  Artificial Sequence
<220>
<223>  Description of Artificial Sequence: Synthesized Em1-reverse primer
<400>  23
acttgcctgt cgctctatct tcgagctgca ttcccaaaca actc                        44

<210>  24
<211>  42
<212>  DNA
<213>  Artificial Sequence
<220>
<223>  Description of Artificial Sequence: Synthesized Pm2-forward primer
<400>  24
tttctgttgg tgctgatatt gcagagtttg atcmtggctc ag                          42

<210>  25
<211>  41
<212>  DNA
<213>  Artificial Sequence
<220>
<223>  Description of Artificial Sequence: Synthesized Pm2-reverse primer
<400>  25
acttgcctgt cgctctatct tcggttacct tgttacgact t                           41

<210>  26
<211>  61
<212>  DNA
<213>  Artificial Sequence
<220>
<223>  Description of Artificial Sequence: Synthesized Adapter Y top
<400>  26
ggcgtctgct tgggtgttta accttttttt tttaatgtac ttcgttcagt tacgtattgc       60
t                                                                       61

<210>  27
<211>  22
<212>  DNA
<213>  Artificial Sequence
<220>
<223>  Description of Artificial Sequence: Synthesized Adapter Y bottom
<400>  27

gcaatacgta actgaacgaa gt 22